(19) **Europäisches Patentamt / European Patent Office / Office européen des brevets**

(11) **EP 4 582 447 A1**

(12) **EUROPEAN PATENT APPLICATION**
published in accordance with Art. 153(4) EPC

(43) Date of publication:
09.07.2025 Bulletin 2025/28

(21) Application number: 23864728.3

(22) Date of filing: 13.09.2023

(51) International Patent Classification (IPC):
$C07K\ 16/22^{(2006.01)}$ $A61K\ 39/395^{(2006.01)}$

(52) Cooperative Patent Classification (CPC):
A61K 39/395; C07K 16/22

(86) International application number:
PCT/CN2023/118600

(87) International publication number:
WO 2024/055996 (21.03.2024 Gazette 2024/12)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 14.09.2022 CN 202211117251

(71) Applicant: Quaerite Biopharm Research (Beijing) Co., Ltd.
Beijing 100195 (CN)

(72) Inventors:
• HUANG, Jinliang
Beijing 100195 (CN)
• LIU, Huiqin
Beijing 100195 (CN)

(74) Representative: HGF
HGF Limited
1 City Walk
Leeds LS11 9DX (GB)

Remarks:
The complete document including Reference Table(s) and the Sequence Listing(s) can be downloaded from the EPO website

(54) **ANTI-VEGFA ANTIBODY OR ANTIGEN-BINDING FRAGMENT THEREOF AND USE THEREOF**

(57) Provided herein are an anti-VEGFA antibody or an antigen-binding fragment thereof and use thereof. The anti-VEGFA antibody or the antigen-binding fragment thereof features high thermal stability, high water solubility, small molecular weight, simple expression and purification processes, and high affinity for VEGFA, and is capable of effectively blocking the VEGFA signaling pathway and effectively blocking VEGFA-induced pathological processes such as vascular endothelial cell proliferation and angiogenesis, thus having potential applications in the treatment of VEGFA-associated diseases.

FIG. 1

EP 4 582 447 A1

## Description

### TECHNICAL FIELD

The present disclosure relates to the technical field of biomedicine, and particularly to an anti-VEGFA antibody or an antigen-binding fragment thereof and use thereof.

### BACKGROUND

Vascular endothelial growth factor A (VEGFA) is a cytokine that is closely associated with angiogenesis. VEGFA promotes vascular endothelial cell proliferation and angiogenesis by binding to its receptors, VEGFR1 and VEGFR2, and activating the downstream signaling pathways of the two receptors.

Pathological angiogenesis occurs in a variety of diseases such as solid tumors, inflammatory responses, and fundus vascular diseases. The tumor cell proliferation in the later stages relies on the provision of sufficient nutrients by new blood vessels to support tumor growth and metastasis. Therefore, inhibition of angiogenesis is an important means for treating cancers. The first anti-VEGFA monoclonal antibody, bevacizumab, was approved in 2004 for the treatment of metastatic colorectal cancer. Overexpression of VEGFA is also closely associated with fundus vascular proliferation, such as in wet age-related macular degeneration (AMD), diabetic macular edema (DME), diabetic retinopathy (DR), etc. Therefore, anti-VEGFA drugs have become the first choice for treating fundus vascular proliferation.

Currently, there are 4 major marketed anti-VEGFA protein drugs for the treatment of wet AMD, including ranibizumab, aflibercept, conbercept, and brolucizumab. Additionally, bevacizumab has also been used off-label for treating wet AMD. The types of these anti-VEGFA protein drugs include traditional monoclonal antibodies, antibody fragments Fab and scFv, and receptor-Fc fusion proteins, but no single domain antibody (variable domain of heavy-chain antibody (VHH)) or nanobody (Nb) drugs are currently approved for marketing.

A single domain antibody or a nanobody is a variable region domain of a heavy-chain antibody found in camelids that naturally lacks light chains, and it is a stable, minimal antibody unit with complete antigen-binding functionality. The single domain antibody has a molecular weight of about 13 kDa, and it features high thermal stability and good water solubility. As a novel antibody form, the nanobody is receiving increasing attention for its development.

Therefore, the present disclosure provides a novel anti-VEGFA antibody or an antigen-binding fragment thereof, which is used for blocking the binding of VEGF to VEGFR1 and VEGR2, thereby further inhibiting the VEGF downstream signaling pathway and inhibiting VEGF-stimulated cell proliferation.

### SUMMARY

In a first aspect of the present disclosure, provided is an anti-VEGFA antibody or an antigen-binding fragment thereof, comprising CDR-H1, CDR-H2, and/or CDR-H3 of a heavy chain variable region.

An amino acid sequence of the CDR-H1 comprises SYTMG (SEQ ID NO: 1) or an amino acid sequence having at least 80% identity to SYTMG (SEQ ID NO: 1);

an amino acid sequence of the CDR-H2 comprises AISKGGYKYX$_1$X$_2$VSLEA (SEQ ID NO: 2) or an amino acid sequence having at least 80% identity to AISKGGYKYX$_1$X$_2$VSLEA (SEQ ID NO: 2);
an amino acid sequence of the CDR-H3 comprises TRAYGSSRLX$_3$LAX$_4$TYEY (SEQ ID NO: 3) or an amino acid sequence having at least 80% identity to TRAYGSSRLX$_3$LAX$_4$TYEY (SEQ ID NO: 3).

X in SEQ ID NO: 2 or SEQ ID NO: 3 may be any natural amino acid residue, such as alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), phenylalanine (F), threonine (T), proline (P), serine (S), tryptophan (W), tyrosine (Y), or valine (V).

In one specific embodiment of the present disclosure, X$_1$X$_2$ in SEQ ID NO: 2 represents DS, DA, NT, DT, NA, or NS; X$_3$ in SEQ ID NO: 3 represents R or K, and X$_4$ in SEQ ID NO: 3 represents D, N, E, or K.

In one specific embodiment of the present disclosure, the amino acid sequences of the CDR-H1, the CDR-H2, and the CDR-H3 comprise any one of the following groups (see Table 1 for details):

A) SEQ ID NOs: 1, 4, and 9;
B) SEQ ID NOs: 1, 5, and 9;
C) SEQ ID NOs: 1, 6, and 10;
D) SEQ ID NOs: 1, 4, and 11;
E) SEQ ID NOs: 1, 7, and 12;

F) SEQ ID NOs: 1, 6, and 11;
G) SEQ ID NOs: 1, 4, and 10;
H) SEQ ID NOs: 1, 5, and 12;
I) SEQ ID NOs: 1, 4, and 12;
J) SEQ ID NOs: 1, 8, and 12; and
K) SEQ ID NOs: 1, 33, and 34.

Table 1. Amino acid sequences of CDR-H1, CDR-H2, and CDR-H3 of candidate antibodies

| Candidate antibody number | SEQ ID NO: x | CDR-H1 | SEQ ID NO: x | CDR-H2 | SEQ ID NO: x | CDR-H3 |
|---|---|---|---|---|---|---|
| V1 | 1 | SYTMG | 4 | AISKGGYKY**DS**VSLEA | 9 | TRAYGSSRL**R**LA**D**TYEY |
| V13 | 1 | SYTMG | 5 | AISKGGYKY**DA**VSLEA | 9 | TRAYGSSRL**R**LA**D**TYEY |
| V14 | 1 | SYTMG | 6 | AISKGGYKY**NT**VSLEA | 10 | TRAYGSSRL**R**LA**N**TYEY |
| V15 | 1 | SYTMG | 4 | AISKGGYKY**DS**VSLEA | 11 | TRAYGSSRL**R**LA**E**TYEY |
| V17 | 1 | SYTMG | 7 | AISKGGYKY**DT**VSLEA | 12 | TRAYGSSRL**R**LA**K**TYEY |
| V21 | 1 | SYTMG | 6 | AISKGGYKY**NT**VSLEA | 11 | TRAYGSSRL**R**LA**E**TYEY |
| V29 | 1 | SYTMG | 4 | AISKGGYKY**DS**VSLEA | 10 | TRAYGSSRL**R**LA**N**TYEY |
| V30 | 1 | SYTMG | 5 | AISKGGYKY**DA**VSLEA | 12 | TRAYGSSRL**R**LA**K**TYEY |
| V31 | 1 | SYTMG | 5 | AISKGGYKY**DA**VSLEA | 12 | TRAYGSSRL**R**LA**K**TYEY |
| V33 | 1 | SYTMG | 4 | AISKGGYKY**DS**VSLEA | 12 | TRAYGSSRL**R**LA**K**TYEY |
| V36 | 1 | SYTMG | 4 | AISKGGYKY**DS**VSLEA | 9 | TRAYGSSRL**R**LA**D**TYEY |
| V40 | 1 | SYTMG | 8 | AISKGGYKY**NA**VSLEA | 12 | TRAYGSSRL**R**LA**K**TYEY |
| V41 | 1 | SYTMG | 4 | AISKGGYKY**DS**VSLEA | 12 | TRAYGSSRL**R**LA**K**TYEY |
| V43 | 1 | SYTMG | 4 | AISKGGYKY**DS**VSLEA | 10 | TRAYGSSRL**R**LA**N**TYEY |
| V19 | 1 | SYTMG | 33 | AISKGGYKY**NS**VSLEA | 34 | TRAYGSSRL**K**LA**N**TYEY |

Preferably, the amino acid sequences of the CDR-H1, the CDR-H2, and the CDR-H3 are arranged in the order from the N terminus to the C terminus, and the amino acids in the CDRs of the antibody in the present application are defined by using the Kabat numbering system.

The anti-VEGFA antibody or the antigen-binding fragment thereof comprises a heavy chain variable region, as well as a CH2 and/or CH3 region.

The structure of the anti-VEGFA antibody or the antigen-binding fragment thereof is a nanobody, a chimeric antibody, a Fab fragment, a Fab' fragment, a Fd fragment, a Fd' fragment, a Fv fragment, a dAb fragment, an isolated CDR, a F(ab')$_2$ fragment, a single domain antibody, a single chain antibody molecule, or a linear antibody.

The anti-VEGFA antibody or the antigen-binding fragment thereof may be a dual-target specific antibody or a specific antibody against three or more targets (e.g., 3, 4, 5, 6, 7, 8, 9, 10 or more, etc.).

The anti-VEGFA antibody or the antigen-binding fragment thereof may be a linear antibody.

The anti-VEGFA antibody or the antigen-binding fragment thereof may be a single domain antibody or a nanobody.

The anti-VEGFA antibody or the antigen-binding fragment thereof may be a humanized antibody or a fully human antibody.

Preferably, the anti-VEGFA antibody or the antigen-binding fragment thereof comprises a humanized sequence, wherein an engineered site of the humanized sequence is located in a non-CDR region, and further preferably, the humanized engineered site is located in a framework region and/or a constant region of the antibody.

In one specific embodiment of the present disclosure, the anti-VEGFA antibody or the antigen-binding fragment thereof is a nanobody, which, at the same mass, has a higher molar concentration and can bind to more antigen molecules as compared to a full-length IgG antibody, Fab, or scFv.

The anti-VEGFA antibody or the antigen-binding fragment thereof can bind to a human or monkey VEGFA protein, wherein the sequence of the human VEGFA protein is identical to that of the monkey VEGFA protein. Preferably, the amino acid sequence of the anti-VEGFA antibody or the antigen-binding fragment thereof comprises any one of the amino acid sequences of SEQ ID NOs: 13-32, 35-61, and 64-67, or has at least 80% identity to any one of the amino acid sequences of SEQ ID NOs: 13-32, 35-61, and 64-67.

In one specific embodiment of the present disclosure, the amino acid sequence of the anti-VEGFA antibody or the antigen-binding fragment thereof is set forth in any one of the amino acid sequences of SEQ ID NOs: 13-32, 35-61, and 64-67.

The anti-VEGFA antibody or the antigen-binding fragment thereof can inhibit other antibodies (preferably those that bind to the same or overlapping epitopes as the anti-VEGFA antibody described in the present disclosure) from or compete with them for binding to a human or monkey VEGFA protein.

The anti-VEGFA antibody or the antigen-binding fragment thereof can be constructed using any conventional method in the prior art, such as artificial synthesis or eukaryotic expression or prokaryotic expression.

In a second aspect of the present disclosure, provided is an anti-VEGFA antibody or an antigen-binding fragment thereof.

The anti-VEGFA antibody or the antigen-binding fragment thereof is a nanobody.

The anti-VEGFA antibody or the antigen-binding fragment thereof comprises any one of the amino acid sequences of SEQ ID NOs: 13-32, 35-61, and 64-67, or has at least 80% identity to any one of the amino acid sequences of SEQ ID NOs: 13-32, 35-61, and 64-67.

In one specific embodiment of the present disclosure, the amino acid sequence of the anti-VEGFA antibody or the antigen-binding fragment thereof is set forth in any one of SEQ ID NOs: 13-32, 35-61, and 64-67.

In a third aspect of the present disclosure, provided is a method for screening the anti-VEGFA antibody or the antigen-binding fragment thereof, which comprises immunizing an alpaca with a human or monkey VEGFA protein.

In a fourth aspect of the present disclosure, provided is a fusion protein comprising the anti-VEGFA antibody or the antigen-binding fragment thereof described above.

Preferably, the fusion protein further comprises, in addition to the anti-VEGFA antibody or the antigen-binding fragment thereof described above, an additional anti-VEGFA antibody or an antibody against an additional target, or an antigen-binding fragment thereof, or an additional functional component.

Preferably, the functional component includes, but is not limited to, one of or a combination of two or more of serum albumin, a cytokine, transferrin, a scaffold protein, an oligopeptide, an oligopeptide polymer, a polypeptide, a polypeptide polymer, a polysaccharide, a fatty chain, avidin, biotin, streptavidin, a toxin, a drug, a nucleic acid, a radionuclide and a marker thereof, a PEGylated ingredient, and a Fc fragment.

Preferably, the additional target is selected from IgG, VEGFB, VEGFC, VEGFD, VEGFR, FGF, FGFR, PlGF, PDGF, ANG2, Endoglin (CD105), TGF, Integrin, Integrin receptors, interleukins (e.g., IL-1β, IL-2, IL-3, IL-4, IL-6, IL-10, IL-12, IL-15, IL-17, IL-23, etc.), interleukin receptors (e.g., IL1R1, IL2Rα, IL3R, IL4Rα, IL6R, IL10R, IL12R, IL15Rα, IL17R, IL23R, etc.), PCSK9, TNF-α, TNFR, RANKL, complement protein C3, complement protein C5, G protein-coupled receptors (GPCRs), GLP1R, CD3, CD19, CD20, CD22, CD25, CD27, CD28, CD30, CD33, CD38, CD40, CD47, CD80, CD86, CD96, CD99, CD111, CD112, CD123, CD133, CD138, CD155, CD171, Claudin 18.2, OX40, ICOS, CTLA4, 4-1BB, TCR, B7-1, B7-2, BTLA, TIM-3, LAG3, Galectin-9, PD-L1, PD-L2, PD-1, TIGIT, EGFR, Her2, PSCA, CEA, FAP, EGFRVIII, BCMA, PSMA, CA125, EphA2, C-met, L1CAM, CS1, ROR1, EC, NY-ESO-1, MUC1, MUC16, mesothelin, LewisY, GPC3, GD2, EPG, DLL 3, and 5T4.

The structure of the antibody or the antigen-binding fragment thereof is a nanobody, a chimeric antibody, a Fab fragment, a Fab' fragment, a Fd fragment, a Fd' fragment, a Fv fragment, a dAb fragment, an isolated CDR, a F(ab')$_2$ fragment, a single domain antibody, a single chain antibody molecule, or a linear antibody. Preferably, the fusion protein comprises at least one anti-VEGFA antibody or antigen-binding fragment thereof. Preferably, the fusion protein comprises at least one additional anti-VEGFA antibody or antigen-binding fragment thereof.

Preferably, the fusion protein comprises at least one antibody against an additional target or antigen-binding fragment thereof, or at least one additional functional component.

The linkage between the anti-VEGFA antibodies or the antigen-binding fragments thereof, between the anti-VEGFA antibody or the antigen-binding fragment thereof and the additional anti-VEGFA antibody or the antigen-binding fragment thereof, between the anti-VEGFA antibody or the antigen-binding fragment thereof and the antibody against the additional target or the antigen-binding fragment thereof or the additional functional component, between the additional anti-VEGFA antibody and the antibody against the additional target or the antigen-binding fragment thereof or the additional functional component, between the antibodies against the additional targets or the antigen-binding fragments thereof or the additional functional components, and between the additional anti-VEGFA antibodies or the antigen-binding fragments thereof, is either direct or indirect.

The indirect linkage may be a linkage via a linker, a functional domain, and/or a linker for coupling. The linker is selected from a linker peptide, an oligopeptide, an oligopeptide polymer, a polypeptide, a polypeptide polymer, PEG, a nucleic acid, a polysaccharide, a fatty chain, biotin, streptavidin, and avidin.

The functional domain is one of or a combination of two or more of a Fc fragment, serum albumin, a cytokine, transferrin, a scaffold protein, an antibody against VEGFA or other targets, and an antigen-binding fragment thereof.

The linker for coupling comprises one of or a combination of two or more of a toxin, a drug, a nucleic acid, PEG, and a radionuclide and a marker thereof.

Preferably, the direct or indirect linkage includes a direct or indirect linkage to the N terminus, C terminus, and/or internal residues of the anti-VEGFA antibody or the antigen-binding fragment thereof, the additional anti-VEGFA antibody, and/or the antibody against the additional target.

The linking order of the anti-VEGFA antibody or the antigen-binding fragment thereof, the additional anti-VEGFA antibody, or the antibody against the additional target comprised in the fusion protein may be such that the N terminus, C terminus, and/or internal residues of one antibody is linked to the N terminus, C terminus, and/or internal residue of another antibody.

In one specific embodiment of the present disclosure, the linking order of the anti-VEGFA antibody or the antigen-binding fragment thereof, the additional anti-VEGFA antibody, or the antibody against the additional target comprised in the fusion protein may be such that the N terminus of one antibody is linked to the N terminus or C terminus of another antibody.

In one specific embodiment of the present disclosure, the linking order of the anti-VEGFA antibody or the antigen-binding fragment thereof, the additional anti-VEGFA antibody, or the antibody against the additional target comprised in the fusion protein may be such that the C terminus of one antibody is linked to the N terminus or C terminus of another antibody.

In a specific embodiment, the fusion protein comprises an anti-VEGFA antibody and a Fc fragment. Preferably, the fusion protein comprises any one of the amino acid sequences of SEQ ID NOs: 64-67, or has at least 80% identity to any one of the amino acid sequences of SEQ ID NOs: 64-67.

Preferably, the fusion protein further comprises a tag.

Preferably, the tag is linked to the C terminus of the fusion protein.

In a fifth aspect of the present disclosure, provided is a chimeric antigen receptor, wherein an extracellular domain of the chimeric antigen receptor comprises the anti-VEGFA antibody or the antigen-binding fragment thereof described above.

Preferably, the chimeric antigen receptor further comprises any transmembrane region and/or intracellular signaling region conventional in the prior art.

In a sixth aspect of the present disclosure, provided is a nucleic acid encoding the anti-VEGFA antibody or the antigen-binding fragment thereof described above or encoding the fusion protein described above or the chimeric antigen receptor described above.

In a seventh aspect of the present disclosure, provided is a vector comprising the nucleic acid described above. The vector is capable of expression *in vivo* or *in vitro* or *ex vivo.* Preferably, the vector is a prokaryotic expression vector, a viral expression vector, or a eukaryotic expression vector, such as an *Escherichia coli* series vector, a phage, etc.

In an eighth aspect of the present disclosure, provided is a host cell comprising the nucleic acid described above or the vector described above.

The host cell may be a eukaryotic cell or a prokaryotic cell.

The eukaryotic cell includes animal and plant cells, such as T cells, yeast cells, HEK293 cells, CHO cells, etc. The prokaryotic cell includes, for example, *Escherichia coli,* etc.

In a ninth aspect of the present disclosure, provided is a preparation method for a host cell, which comprises introducing the nucleic acid or the vector described above into a host cell, and subsequently inducing expression thereof.

In a tenth aspect of the present disclosure, provided is a preparation method for the anti-VEGFA antibody or the antigen-binding fragment thereof, which comprises introducing a nucleic acid or vector encoding the anti-VEGFA antibody or the antigen-binding fragment thereof into a host cell, and subsequently inducing expression thereof.

In an eleventh aspect of the present disclosure, provided is an immune cell expressing the anti-VEGFA antibody or the antigen-binding fragment thereof described above or the chimeric antigen receptor described above. Preferably, the immune cell includes, but is not limited to, lymphocytes (e.g., T cells, B cells, and NK cells), dendritic cells, monocytes/macrophages, granulocytes, and mast cells.

Preferably, the immune cell is a CAR-immune cell.

In a twelfth aspect of the present disclosure, provided is a method for constructing an immune cell, which comprises transfecting an immune cell with a nucleic acid sequence encoding the chimeric antigen receptor described in the present disclosure for expression.

In a thirteenth aspect of the present disclosure, provided is a product for treating and/or diagnosing a disease, which comprises any one of the following:

A) the anti-VEGFA antibody or the antigen-binding fragment thereof described above;

B) the fusion protein described above;

C) the chimeric antigen receptor described above;
D) the immune cell described above;
E) the nucleic acid described above;
F) the vector described above; or
G) the host cell described above.

The product for treating and/or diagnosing a disease targets cells expressing VEGFA, wherein the cells may be cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, or the like.

Preferably, the product may be a kit or a medicament or a chip or an antibody-drug conjugate, or the like. The disease is a disease associated with the VEGFA signaling pathway. Further preferably, it may be a tumor, abnormal vascular proliferation, an ophthalmic disease involving angiogenesis (e.g., a fundus vascular disease), or the like.

In a fourteenth aspect of the present disclosure, provided is an antibody-drug conjugate (ADC), which comprises the anti-VEGFA antibody or the antigen-binding fragment thereof described in the present disclosure that is covalently bound to a drug.

In a fifteenth aspect of the present disclosure, provided is a method for detecting VEGFA, which comprises contacting a sample to be tested with the anti-VEGFA antibody or the antigen-binding fragment thereof described above, and subsequently detecting the content of a complex formed by VEGFA and the anti-VEGFA antibody or the antigen-binding fragment thereof.

The detection method is to detect the existence or the content of VEGFA. The existence indicates presence or absence, and the content may be an expression level, a protein concentration, or the like.

In a sixteenth aspect of the present disclosure, provided is a method for diagnosing a disease, which comprises taking a sample, contacting the sample with the anti-VEGFA antibody or the antigen-binding fragment thereof, the chimeric antigen receptor described above, the fusion protein described above, the nucleic acid described above, the vector described above, the host cell described above, the immune cell described above, or the product for treating and/or diagnosing a disease described above, and detecting the content of a complex formed by VEGFA and the anti-VEGFA antibody or the antigen-binding fragment thereof.

The disease is a disease associated with the VEGFA signaling pathway. Further preferably, it may be a tumor, abnormal vascular proliferation, an ophthalmic disease involving angiogenesis (e.g., a fundus vascular disease), or the like.

In a seventeenth aspect of the present disclosure, provided is a method for treating and/or preventing a disease, which comprises administering to an individual the anti-VEGFA antibody or the antigen-binding fragment thereof described above, the chimeric antigen receptor described above, the fusion protein described above, the nucleic acid described above, the vector described above, the host cell described above, the immune cell described above, or the product for treating and/or diagnosing a disease described above.

The disease is a disease associated with the VEGFA signaling pathway. Further preferably, it may be a tumor, abnormal vascular proliferation, an ophthalmic disease involving angiogenesis (e.g., a fundus vascular disease), or the like.

In an eighteenth aspect of the present disclosure, provided is a method for blocking VEGFA-mediated vascular endothelial cell proliferation or inhibiting angiogenesis, which comprises contacting a vascular endothelial cell with the anti-VEGFA antibody or the antigen-binding fragment thereof described above, the chimeric antigen receptor described above, the fusion protein described above, the nucleic acid described above, the vector described above, the host cell described above, the immune cell described above, or the product for treating and/or diagnosing a disease described above.

Preferably, the method comprises diluting the anti-VEGFA antibody or the antigen-binding fragment thereof described above, the chimeric antigen receptor described above, the fusion protein described above, the nucleic acid described above, the vector described above, the host cell described above, the immune cell described above, or the product for treating and/or diagnosing a disease described above with a serum-free medium of vascular endothelial cells, followed by incubation with an antigen (e.g., VEGFA165).

Preferably, the step of discarding the vascular endothelial cell complete medium in the culture plate after the incubation is comprised.

Preferably, the antigen and the anti-VEGFA antibody or the antigen-binding fragment thereof described above, the chimeric antigen receptor described above, the fusion protein described above, the nucleic acid described above, the vector described above, the host cell described above, the immune cell described above, or the product for treating and/or diagnosing a disease described above are added to a vascular endothelial cell culture plate and cultured on the culture plate.

Preferably, a detection is performed after the culture.

Preferably, the incubation is performed for 0.5-5 h, preferably 1-3 h, e.g., 0.5 h, 1 h, 2 h, 3 h, 4 h, or 5 h. Preferably, the incubation is performed at a temperature of room temperature to 45 °C, preferably 30-40 °C, e.g., 25 °C, 30 °C, 35 °C, 36 °C, 37 °C, 38 °C, 39 °C, 40 °C, 45 °C, etc.

Preferably, the culture is performed at a temperature of room temperature to 45 °C, preferably 30-40 °C, e.g., 25 °C, 30 °C, 35 °C, 36 °C, 37 °C, 38 °C, 39 °C, 40 °C, 45 °C, etc. Preferably, the culture is performed in an incubator with 5% $CO_2$.

Preferably, the culture is performed for 1-5 days, preferably 2-4 days, e.g., 1 day, 1.5 days, 2 days, 2.5 days, 3 days, 2.5 days, 4 days, 4.5 days, 5 days, etc.

Preferably, the detection is to detect the number of viable vascular endothelial cells.

In a nineteenth aspect of the present disclosure, provided is a method for treating and/or preventing a disease, which comprises contacting the anti-VEGFA antibody or the antigen-binding fragment thereof described above, the chimeric antigen receptor described above, the fusion protein described above, the nucleic acid described above, the vector described above, the host cell described above, the immune cell described above, or the product for treating and/or diagnosing a disease described above with a target cell.

The disease is a disease associated with the VEGFA signaling pathway. Further preferably, it may be a tumor, abnormal vascular proliferation, an ophthalmic disease involving angiogenesis (e.g., a fundus vascular disease), or the like.

Preferably, the target cell is selected from cells expressing VEGFA, such as cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, etc.

In a twentieth aspect of the present disclosure, provided is use of the anti-VEGFA antibody or the antigen-binding fragment thereof described above, the chimeric antigen receptor described above, the fusion protein described above, the nucleic acid described above, the vector described above, the host cell described above, or the immune cell described above in preparing a product for treating and/or preventing a VEGFA-associated disease, or in preparing a product for blocking VEGFA-mediated vascular endothelial cell proliferation or inhibiting angiogenesis, or in preparing an antibody-drug conjugate or an antibody diagnostic kit or a tracer.

The disease may be a tumor, abnormal vascular proliferation, an ophthalmic disease involving angiogenesis (e.g., a fundus vascular disease), or the like.

The product may be a kit or a medicament or a chip or an antibody-drug conjugate, or the like.

The present disclosure provides a novel anti-VEGFA antibody or an antigen-binding fragment thereof, which features high thermal stability, high water solubility, small molecular weight, simple expression and purification processes, and high affinity for VEGFA, and is capable of effectively blocking the VEGFA signaling pathway and effectively blocking VEGFA-induced pathological processes such as vascular endothelial cell proliferation, angiogenesis, etc., making it potentially applicable in the treatment of VEGFA-associated diseases. It may also bring greater clinical value, such as better tissue penetration, lower production costs, more convenient modes of administration, etc.

The "medicament" described in the present disclosure may be used for treating a human or non-human animal, such as a non-human mammal. The medicament may comprise pharmaceutically acceptable carriers, auxiliary materials or salts common in the prior art. The medicament may be administered by any suitable route of administration, such as gastrointestinal routes (e.g., oral administration) or parenteral routes (e.g., intravenous, intramuscular, subcutaneous, intradermal, intraorgan, intranasal, intraocular, instillation, intracerebral, intrathecal, transdermal, intrarectal administrations, etc.). The medicament may be in any suitable dosage form, such as dosage forms for gastrointestinal or parenteral administration. The dosage forms preferably include, but are not limited to, tablets, pills, powders, granules, capsules, lozenges, syrups, liquids, emulsions, microemulsions, suspensions, injections, sprays, aerosols, powder aerosols, lotions, ointments, plasters, pastes, patches, eye drops, nose drops, sublingual tablets, suppositories, aerosols, effervescent tablets, dripping pills, gels, and the like. The various dosage forms of the medicament can be prepared according to conventional production methods in the field of pharmaceuticals. The medicament may comprise the anti-VEGFA antibody or the antigen-binding fragment thereof, the nucleic acid, the vector, the host cell, the immune cell, and the like in a weight ratio of 0.01%-99.5% (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99%, or 99.5%). The medicament may be prepared as a reagent with a protein concentration of 1-300 mg/mL (e.g., 1 mg/mL, 5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL). A single administration dose of the medicament may be 0.1-1000 mg, e.g., 0.1 mg, 0.2 mg, 0.5 mg, 0.75 mg, 1 mg, 1.25 mg, 1.5 mg, 1.75 mg, 2 mg, 2.25 mg, 2.5 mg, 3 mg, 5 mg, 10 mg, 20 mg, 50 mg, 80 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg.

The "pharmaceutically acceptable" described in the present disclosure refers to the biological activity and properties of neither significantly stimulating an organism nor inhibiting the active substance of the administered product.

The "method for..." described in the present disclosure can be used for the purposes of diagnosing and treating diseases or non-disease conditions.

The "antigen-binding fragment" described in the present disclosure is a portion of an antibody that retains the specific binding activity of the antibody, i.e., any portion of the antibody that is capable of specifically binding to an epitope on a target molecule of the antibody. It includes, for example, Fab, Fab', F(ab')$_2$, Fv, Fd, and variants of these fragments, such as the heavy and/or light chain of the antibody, the heavy and/or light chain variable region of the antibody, or a single or 2 or more CDRs from the heavy or light chain of the antibody. Among these, Fab is a monovalent fragment consisting of VL, VH, CL, and CH1 domains. F(ab')$_2$ is a bivalent fragment comprising two Fab fragments linked by disulfide bonds at the hinge region. Fd is a Fd fragment consisting of VH and CH1 domains. Fv is a Fv fragment consisting of VL and VH domains of a single arm of an antibody. Fab' is a Fab fragment with one or more cysteine residues at the C terminus of the CH1 domain. Fab'-SH is a Fab' in which the cysteine residue(s) of the constant domain bear at least one free thiol group. VH represents a heavy chain variable region, VL represents a light chain variable region, and CL represents a light chain.

The "CH2" or "CH3" described in the present disclosure is the CH2 or CH3 of the heavy chain constant region. A complete heavy chain constant region consists of three domains, namely CH1, CH2, and CH3. Specifically, the CH2 domain refers to the portion of an antibody heavy chain polypeptide that extends from about EU position 231 to EU position 340 (according to the EU numbering system of Kabat). The CH2 domain is unique in that it does not pair closely with another domain. The CH3 domain refers to the portion of an antibody heavy chain polypeptide that extends approximately from EU position 341 to EU position 446.

The "Fc" region described in the present disclosure comprises two heavy chain fragments containing the CH2 and CH3 domains of the antibody. The two heavy chain fragments form a dimer through two or more disulfide bonds in the hinge region and are held together by the hydrophobic interactions in the CH3 domains.

The "linear antibody" described in the present disclosure comprises a pair of tandem Fd segments (VH-CH1-VH-CH1).

The "comprises" or "comprising" described in the present disclosure is an open-ended expression. When the term is used to describe a sequence of a protein or nucleic acid, the protein or the nucleic acid may consist of the sequence, or may have additional amino acids or nucleotides at one or both ends of the protein or the nucleic acid, while still retaining the same or similar activity as the original sequence.

The "homology" described in the present disclosure means that, in the case of using protein sequences or nucleotide sequences, those skilled in the art can adjust the sequences according to the actual work needs, such that the sequences used have (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% homology to the sequences obtained with the prior art.

The "humanized antibody" described in the present disclosure refers to an antibody whose framework regions and/or constant regions (e.g., CH regions) are encoded by human antibody genes or an antibody that is entirely encoded by human antibody genes. In one specific embodiment of the present disclosure, the CDRs of the antibody are not engineered.

The "individual" described in the present disclosure may be a human or a non-human mammal, and the non-human mammal may be a wild animal, a zoo animal, an economic animal, a companion animal, a laboratory animal, or the like. Preferably, the non-human mammal includes, but is not limited to, a pig, a cow, a sheep, a horse, a donkey, a fox, a raccoon dog, a mink, a camel, a dog, a cat, a rabbit, a rodent (e.g., a rat, a mouse, a guinea pig, a hamster, a gerbil, a chinchilla, and a squirrel), a monkey, or the like.

The "treating" described in the present disclosure refers to slowing, interrupting, arresting, controlling, stopping, reducing, or reversing the progression or severity of a sign, symptom, disorder, condition, or disease after the disease has begun to progress, but it does not necessarily involve the complete elimination of all disease-related signs, symptoms, conditions, or disorders.

The "preventing" described in the present disclosure refers to the practice of preventing or delaying the onset of a disease or condition or symptom in the body.

The "diagnosing" described in the present disclosure refers to the determination of whether a patient has suffered from, is suffering from, or will suffer from a disease or condition in the past, at the time of diagnosis, or in the future, or the determination of the progression or likely progression in the future of a disease.

The tumor described in the present disclosure may be any adverse cell proliferation (or any disease that manifests itself as adverse cell proliferation) or neoplasm, or increased predisposition or risk for adverse cell proliferation, neoplasm or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph nodes, lymphoblasts, maxilla, mediastinum, mesenterium, myometrium, nasopharynx, omentum, oral cavity, ovary, pancreas, parotid gland, peripheral nervous system, peritoneum, pleura, prostate, salivary glands, sigmoid colon, skin, small intestine, soft tissue, spleen, stomach, testis, thymus, thyroid, tongue, tonsils, trachea, uterus, vulva, and white blood cells. Further preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, rhabdomyosarcoma, tongue squamous cell carcinoma, nasopharyngeal cancer, ovarian cancer, placental choriocarcinoma, lymphoma (e.g., non-Hodgkin's lymphoma, Hodgkin's lymphoma, and cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic granulocytic leukemia, acute myelogenous leukemia, myelodysplastic syndrome, chronic lymphocytic leukemia, epidermoid cancer, colon cancer, thymus cancer, blood cancer, head and neck cancer, and oropharyngeal cancer.

The "fundus vascular disease" described in the present disclosure refers to the general term for diseases occurring in the retinal arteries or veins or diseases associated with choroidal neovascularization, including but not limited to age-related macular degeneration (AMD), diabetic macular edema (DME), diabetic retinopathy, central retinal vein occlusion, pathological myopia, neovascular glaucoma, and the like.

## BRIEF DESCRIPTION OF THE DRAWINGS

Hereinafter, examples of the present disclosure will be described in detail with reference to the accompanying drawings, in which:

FIG. 1: Coomassie-stained gel images of candidate antibodies purified in different batches.

FIG. 2: Binding signal of candidate antibodies to VEGFA versus antibody concentration.

FIG. 3: Competitive ELISA results of different candidate antibodies.

FIG. 4: Inhibition rates of HUVEC proliferation by different candidate antibodies.

FIG. 5: ELISA detection of the binding activity of antibody V1 to human VEGFA121.

FIG. 6: ELISA detection of the binding activity of antibody V1 to mouse VEGFA164.

FIG. 7: ELISA detection of the binding activity of antibody V1 to rat VEGFA164.

FIG. 8: Coomassie-stained gel images of humanized antibodies after purification.

FIG. 9: ELISA results of humanized antibodies binding to VEGFA.

FIG. 10: ELISA results of humanized antibodies competing with VEGFR2.

FIG. 11: ELISA results of humanized antibodies V30 and V43 competing with VEGFR2.

FIG. 12: Binding and dissociation curves (SPR) of V1-SA1 with VEGFA165.

FIG. 13: Melting temperatures ($Tm$) of different humanized antibodies.

FIG. 14: SDS-PAGE gel image of humanized antibody V1-SA1 and positive control antibody BI-VEGF ab after purification.

FIG. 15: Coomassie-stained gel images of monovalent nanobody-Fc fusion proteins after purification.

FIG. 16: ELISA results of monovalent nanobody-Fc fusion proteins binding to VEGFA.

FIG. 17: Binding and dissociation curves (SPR) of V1-SA1-Fc-m1 with VEGFA165.

## DETAILED DESCRIPTION

The technical solutions in the examples of the present disclosure will be described clearly and completely below with reference to the drawings. It is apparent that the described examples are only a part of the examples of the present disclosure, but not all of them. Based on the examples of the present disclosure, all other examples obtained by those of ordinary skill in the art without creative work shall fall within the protection scope of the present disclosure.

### Example 1: Alpaca Immunization and Antibody Primary Screening

#### 1. Alpaca immunization

Human VEGFA165 (label-free, purchased from Sino Biological, Cat. No.: HPLC-10008-HNAH, hereinafter referred to as VEGFA) was emulsified and subsequently used to immunize alpacas. Two alpacas were selected, and each animal was immunized with 1 mg of VEGFA protein once every 2 weeks. Starting from the second immunization, serum was taken for potency detection. Among them, alpaca 1# was immunized with the antigen 4 times, and alpaca 2# was immunized with the antigen 3 times. Through serum potency detection, both were found to meet the standards for library construction.

#### 2. Detection for competitive activity of post-immunization serum in blocking binding of VEGFA to VEGFR2

The extracellular domain of the VEGF receptor, VEGFR2-ECD, was biotinylated (designated as VEGFR2-biotin), and VEGFA was diluted to 0.5 μg/mL with a CBS buffer and then used to coat a microplate at 4 °C overnight. After being blocked with 3% skim milk powder, the plate was washed. The alpaca serum and the negative serum (the alpaca serum before antigen immunization) were each diluted 5-fold, 15-fold, 45-fold, 135-fold, 405-fold, 1215-fold, and 3645-fold in a VEGFR2-biotin solution with a final concentration of 0.5 μg/mL. Next, 100 μL of each dilution was added to the wells that had been blocked, and the plate was left to stand at room temperature for 1 h. The plate was then washed 3 times with PBST (PBS containing 0.1% Tween 20, pH 7.4), and streptavidin-HRP was added. The plate was left to stand at room temperature for 1 h and then washed 3 times with PBST. Subsequently, TMB was added for color development for 15 min, followed by the addition of a stopping solution, and the absorbance at 450 nm was read using a microplate reader. The detection results for the competitive activity of the sera from the two alpacas are shown in Tables 2-3.

Table 2. Detection results for competitive activity of serum from alpaca 1#

| Alpaca 1# | Serum dilution factor | Coated with VEGFA at 0.5 μg/mL | | |
|---|---|---|---|---|
| | | Immunized serum | Negative serum | Competition rate |
| With VEGFR2-biotin at 0.5 μg/mL added simulta- neously | 5 × | 0.0824 | 0.182 | 54.73% |
| | 15 × | 0.1678 | 0.315 | 46.73% |
| | 45 × | 0.2781 | 0.3672 | 24.26% |
| | 135 × | 0.3456 | 0.3998 | 13.56% |
| | 405 × | 0.3568 | 0.3988 | 10.53% |
| | 1215 × | 0.3922 | 0.3902 | -0.51% |
| | 3645 × | 0.4197 | 0.4106 | -2.22% |

Table 3. Detection results for competitive activity of serum from alpaca 2#

| Alpaca 2# | Serum dilution factor | Coated with VEGFA at 0.5 $\mu$g/mL | | |
|---|---|---|---|---|
| | | Immunized serum | Negative serum | Competition rate |
| With VEGFR2-biotin at 0.5 $\mu$g/mL added simultaneously | 5 $\times$ | 0.0544 | 0.2004 | 72.85% |
| | 15 $\times$ | 0.0874 | 0.3281 | 73.36% |
| | 45 $\times$ | 0.1383 | 0.3904 | 64.57% |
| | 135 $\times$ | 0.2063 | 0.3635 | 43.25% |
| | 405 $\times$ | 0.2862 | 0.411 | 30.36% |
| | 1215 $\times$ | 0.3569 | 0.4003 | 10.84% |
| | 3645 $\times$ | 0.3862 | 0.4273 | 9.62% |

The results demonstrate that antibodies blocking the binding of VEGFA to VEGFR2 were detected in both alpaca #1 and alpaca #2, which can be used for library construction.

### 3. Antibody library construction

40 mL of alpaca peripheral blood was drawn using a blood collection needle, and the red blood cells were lysed according to the instructions of the red blood cell lysis solution. Subsequently, the white blood cells were collected, and trizol was added for cryopreservation. Before library construction, total RNA was extracted from the isolated alpaca PBMCs using trizol and reverse-transcribed into cDNA. The variable region VH fragments were amplified with a nanobody-specific primer. The fragments obtained above and the pcomb3X vector were digested with SfiI enzyme, and after digestion, they were mixed in an appropriate proportion and ligated using a T4 ligase. After ligation, the constructs were used for electroporation of XL1-Blue competent cells. Based on colony growth, the transformation library capacities of the 2 alpacas were calculated to be $6.52 \times 10^8$ and $4.12 \times 10^8$, respectively. Randomly selected clones from the transformant colonies were sent for sequencing validation, and the sequencing results showed that the clones were the correct nanobody sequences.

### 4. Antibody library screening

The phage display method was utilized to screen for phages capable of binding to VEGFA.

For the alpaca 1# library, after 4 rounds of enrichment screening, one 96-well plate of monoclonal colonies (the screening method was recorded as VEGF-4th enrichment) was picked, and the binding activity of the phages to VEGFA was detected. Meanwhile, competitive elution was performed on the phages from the fourth round: 10 $\mu$g/mL VEGFR2 extracellular domain VEGFR2-ECD-His (prepared in-house) was used to competitively elute the phages bound to VEGF, with the expectation of obtaining antibodies that could compete with VEGFR2 for binding to VEGFA. Five 96-well plates of the obtained phages were picked, and the binding activity of the phages to VEGFA was detected (the screening method was recorded as VEGF-5th VEGFR2 competition). In addition, the phages obtained from the VEGFR2 competition screening were further subjected to competitive elution screening with the control monoclonal antibody bevacizumab (Avastin). Specifically, 10 $\mu$g/mL bevacizumab was used to competitively elute the phages bound to VEGFA. One 96-well plate of the obtained phages was picked, and the binding activity of the phages to VEGFA was detected (the screening method was recorded as VEGF-6th bevacizumab competition). The detection of phages was carried out using an HRP-labeled antibody that recognizes the phage coat protein, labeled as anti-M13(HRP). The detection results of the positive clones with VEGFA binding activity among the monoclonal colonies described above are shown in Table 4.

Table 4. Detection results for positive clones with VEGFA binding activity after screening of alpaca 1# library

| | Secondary antibody: anti-M13(HRP) | | |
| --- | --- | --- | --- |
| | Coating: VEGFA protein at 0.5 μg/mL | Coating: 3% milk powder (negative control) | |
| Candidate antibody number | OD450 | | Screening method |
| V1 | 2.3102 | 0.0377 | VEGF-4th enrichment |
| V2 | 2.444 | 0.0233 | |
| V4 | 1.4967 | 0.0369 | VEGF -5th VEGFR2 competition-1# plate |
| V5 | 1.3397 | 0.0223 | |
| V6 | 1.3564 | 0.0473 | |
| V7 | 1.6891 | 0.0229 | VEGF -5th VEGFR2 competition-3# plate |
| V8 | 0.9241 | 0.0236 | |
| V9 | 1.6515 | 0.0457 | VEGF -5th VEGFR2 competition-4# plate |
| V10 | 1.6432 | 0.0449 | |
| V12 | 1.0286 | 0.0413 | VEGF -5th VEGFR2 competition-5# plate |
| V13 | 2.1417 | 0.0144 | VEGF-6th bevacizumab competition |
| V14 | 2.034 | 0.0252 | |
| V15 | 1.8694 | 0.0126 | |
| V16 | 1.3892 | 0.0108 | |
| V17 | 3.473 | 0.0403 | |
| V18 | 2.9355 | 0.0134 | |
| V19 | 2.4741 | 0.0273 | |
| V20 | 2.3907 | 0.0171 | |
| V21 | 2.1493 | 0.0186 | |
| V22 | 1.637 | 0.0353 | |
| V23 | 1.4319 | 0.0163 | |
| V24 | 1.3692 | 0.0193 | |
| V25 | 1.2943 | 0.0354 | |
| V26 | 1.1503 | 0.0215 | |
| V27 | 0.8976 | 0.0127 | |
| V28 | 0.4648 | 0.0128 | |

In addition, biotinylated VEGFA was used to screen for phages that bind to more epitopes, with the method being as follows: Avidin was used for coating, followed by incubation with biotinylated VEGFA. After the alpaca 1# library was merged with the alpaca 2# library, phages that bind to VEGFA were obtained through the incubation of the merged library with the biotinylated VEGFA and elution. This process was repeated. After multiple rounds of enrichment, one 96-well plate of monoclonal colonies was selected. The phages in the supernatant were subjected to ELISA to detect the binding activity to VEGFA, and the results for the positive clones with binding activity are shown in Table 5.

Table 5. Detection results for screening for positive clones with VEGFA binding activity after library merging

| | Secondary antibody: anti-M13(HRP) | |
| --- | --- | --- |
| | Coating: VEGFA protein at 0.5 µg/mL | Coating: 3% milk powder (negative control) |
| Candidate antibody number | OD450 | |
| V29 | 2.6876 | 0.0553 |
| V30 | 2.6791 | 0.0436 |
| V31 | 2.6716 | 0.0363 |
| V32 | 2.2859 | 0.0494 |
| V33 | 2.5274 | 0.0489 |
| V34 | 2.3355 | 0.057 |
| V35 | 2.6665 | 0.0549 |
| V36 | 2.7688 | 0.0623 |
| V37 | 2.7778 | 0.0397 |
| V38 | 2.8454 | 0.0419 |
| V39 | 3.2891 | 0.0359 |
| V40 | 3.1083 | 0.0362 |
| V41 | 2.7719 | 0.0359 |
| V42 | 2.6942 | 0.0691 |
| V43 | 2.5025 | 0.0519 |

## 5. Primary screening for antibody sequences

The phages with VEGFA binding activity described above were subjected to plasmid sequencing and codon translation to obtain the amino acid sequences of candidate nanobodies (CDRs + framework regions). Antibodies with identical sequences were merged, and the candidate antibodies with different CDR sequences are shown in Table 6.

Table 6. Amino acid sequences of candidate antibodies

| SEQ ID NO: x | Candidate antibody number | Antibody amino acid sequence |
| --- | --- | --- |
| 13 | V1 | QLQLVESGGGSVQPGGSLRLSCEVSGRTFSSYTMGWFRQAPGK EREFVVAISKGGYKYDSVSLEARFTISKDNTKNTVYLQMNSLK PEDTAVYYCAATRAYGSSRLRLADTYEYWGQGTQVTVSS |
| 14 | V6 | QLQLVESGGGSVQPGGSLRLSCEVSGRTFSSYTMGWFRQAPGK EREFVVAISKGGYKYDTVSLEARFTISKDNTKNTVYLQMNSLK PEDTAVYYCAGTRAYGSSRLKLADTYEYWGQGTQVTVSS |
| 15 | V8 | QLQLVESGGGSVQPGGSLRLSCEVSGRTFSSYTMGWFRQAPGK EREFVVAISKGGYKYNTVSLEARFTISKDNTKNTVYLQMNSLK PEDTAVYYCAGTRAYGSSRLKLAETYEYWGQGTQVTVSS |
| 16 | V13 | QLQLVESGGGSVQPGGSLRLSCEVSGRTFTSYTMGWFRQAPGK EREFVVAISKGGYKYDAVSLEARFTISKDNTKNTVYLQMNSLK PEDTAVYYCAGTRAYGSSRLRLADTYEYWGQGTQVTVSS |

(continued)

| SEQ ID NO: x | Candidate antibody number | Antibody amino acid sequence |
|---|---|---|
| 17 | V14 | QLQLVESGGGSVQPGGSLRLSCEVSGRTFTSYTMGWFRQAPGK EREFVVAISKGGYKYNTVSLEARFTISKDNTKNTVYLQMNSLK PEDTAVYYCAGTRAYGSSRLRLANTYEYWGQGTQVTVSS |
| 18 | V15 | QLQLVESGGGSVQPGGSLRLSCEVSGRTFASYTMGWFRQAPGK EREFVVAISKGGYKYDSVSLEARFTISKDNTKNTVYLQMNSLK PEDTAVYYCAGTRAYGSSRLRLAETYEYWGQGTQVTVSS |
| 19 | V17 | QLQLVESGGGSVQPGGSLRLSCEVSGRTFSSYTMGWFRQAPGK EREFVVAISKGGYKYDTVSLEARFTISKDNTKNTVYLQMNSLK PEDTAVYYCAATRAYGSSRLRLAKTYEYWGQGTQVTVSS |
| 20 | V18 | QLQLVESGGGSVQPGGSLRLSCEVSGRTFSSYTMGWFRQAPGK EREFVVAISKGGYKYNSVSLEARFTISKDNTKNTVYLQMNSLK PEDTAVYYCAGTRAYGSSRLRLADTYEYWGQGTQVTVSS |
| 21 | V19 | QLQLVESGGGSVQPGGSLRLSCEVSGRTFSSYTMGWFRQAPGK EREFVVAISKGGYKYNSVSLEARFTISKDNTKNTVYLQMNSLK PEDTAVYYCAATRAYGSSRLKLANTYEYWGQGTQVTVSS |
| 22 | V21 | QLQLVESGGGSVQPGGSLRLSCEVSGRTFTSYTMGWFRQAPGK EREFVVAISKGGYKYNTVSLEARFTISKDNTKNTVYLQMNSLK PEDTAVYYCAGTRAYGSSRLRLAETYEYWGQGTQVTVSS |
| 23 | V22 | QLQLVESGGGSVQPGGSLRLSCEVSGRTFSSYTMGWFRQAPGK EREFVVAISKGGYKYDSVSLEARFTISKDNTKNTVYLQMNSLK PEDTAVYYCAATRAYGSSRLKLADTYEYWGQGTQVTVSS |
| 24 | V25 | QLQLVESGGGSVQPGGSLRLSCEVSGRTFTSYTMGWFRRAPGK EREFVVAISKGGYKYDSVSLEARFTISKDNTKNTVYLQMNSLK PEDTAVYYCAGTRAYGSSRLKLAKTYEYWGQGTQVTVSS |
| 25 | V29 | QLQLVESGGGSVQPGGSLRLSCEVSGRTFSSYTMGWFRQAPGK EREFVVAISKGGYKYDSVSLEARFTISKDNTKNTVYLQMNSLK PEDTAVYYCAGTRAYGSSRLRLANTYEYWGQGTQVTVSS |
| 26 | V30 | QLQLVESGGGSVQPGGSLRLSCEVSGRTFSSYTMGWFRQAPGK GREFVVAISKGGYKYDAVSLEARFTISKDNTKNTVYLQMNSLK PEDTAVYYCAATRAYGSSRLRLAKTYEYWGQGTQVTVSS |
| 27 | V31 | QLQLVESGGGSVQPGGSLRLSCEVSGRTFASYTMGWFRQAPGK EREFVVAISKGGYKYDAVSLEARFTISKDNTKNTVYLQMNSLK PEDTAVYYCAGTRAYGSSRLRLAKTYEYWGQGTQVTVSS |
| 28 | V33 | QLQLVESGGGSVQPGGSLRLSCEVSGRTFSSYTMGWFRQAPGK EREFVVAISKGGYKYDSVSLEARFTISKDNTKNTVYLQMNSLK PEDTAVYYCAATRAYGSSRLRLAKTYEYWGQGTQVTVSS |

(continued)

| SEQ ID NO: x | Candidate antibody number | Antibody amino acid sequence |
|---|---|---|
| 29 | V36 | QLQLVESGGGSVQPGGSLRLSCEVSGRTFASYTMGWFRQAPGK EREFVVAISKGGYKYDSVSLEARFTISKDNTKNTVYLQMNSLK PEDTAVYYCAGTRAYGSSRLRLADTYEYWGQGTQVTVSS |
| 30 | V40 | QLQLVESGGGSVQPGGSLRLSCEVSGRTFTSYTMGWFRQAPGK EREFVVAISKGGYKYNAVSLEARFTISKDNTKNTVYLQMNSLK PEDTAVYYCAGTRAYGSSRLRLAKTYEYWGQGTQVTVSS |
| 31 | V41 | QLQLVESGGGSVQPGGSLRLSCEVSGRTFSSYTMGWFRQAPGK EREFVVAISKGGYKYDSVSLEARFTISKDNTKNTVYLQMNSLK PEDTAVYYCAGTRAYGSSRLRLAKTYEYWGQGTQVTVSS |
| 32 | V43 | QLQLVESGGGSVQPGGSLRLSCEVSGRTFTSYTMGWFRQAPGK EREFVVAISKGGYKYDSVSLEARFTISKDNTKNTVYLQMNSLK PEDTAVYYCAGTRAYGSSRLRLANTYEYWGQGTQVTVSS |

**6. Expression and purification of candidate antibodies**

Some of the candidate antibodies in the table above were selected, expressed using mammalian cells, purified, and then subjected to ELISA to repeatedly validate the VEGFA binding activity of the antibodies.

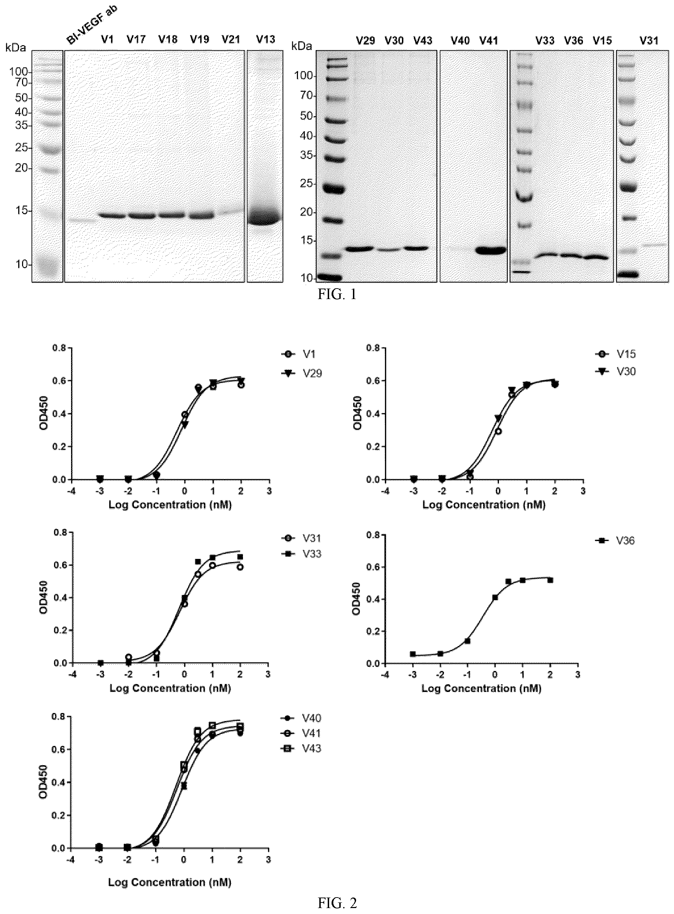

The coding gene of each nanobody was amplified from an original phage plasmid by the PCR method and constructed into a pVRC8400 expression vector. A secretion peptide of MDAMKRGLCCVLLLCGAVFVSPS (SEQ ID NO: 68) was added to the N terminus of the antibody, while a flexible linker GGGGS (SEQ ID NO: 69) and a 6×His tag were sequentially added to the C terminus of the antibody. Meanwhile, the coding gene sequence of the positive control antibody BI-VEGF ab (the antibody is a nanobody against VEGFA, and the amino acid sequence of the antibody is derived from SEQ ID NO: 57 in the patent US 9527925 B2) was synthesized, and a 6×His tag was added to the C terminus of the control antibody BI-VEGF ab. After the expression plasmids for the antibodies were confirmed correct by sequencing, large-scale endotoxin-free extraction was performed. Polyethylenimine (abbreviated as PEI, linear, MW = 25 kDa, purchased from Polysciences, Cat. No.: 23966-1), a transfection reagent, was used to perform transient transfection on 293F suspension cells. The cell supernatant was collected on day 4 or day 5 after transfection, and Ni-bead affinity purification was carried out. The purified proteins were analyzed by SDS-PAGE, and the molecular weight was in line with the expected value (about 15 kDa). The Coomassie-stained gel images of the positive control antibody BI-VEGF ab and some of the candidate antibodies after purification are shown in FIG. 1.

**Example 2: Antibody Activity Assay**

**1. Assay for binding activity of candidate antibodies to VEGFA**

Antigen VEGFA was diluted with ELISA coating buffer (final concentration of 0.3 μg/mL) and added to a microplate at 100 μL/well for coating at 4 °C overnight. After the plate was blocked with 5% skim milk powder, the positive control antibody BI-VEGF ab (the antibody is a nanobody against VEGFA, and the amino acid sequence of the antibody is derived from SEQ ID NO: 57 in the patent US 9527925 B2; the construction method of its expression plasmid as well as the protein purification method are the same as those described in "6. Expression and purification of candidate antibodies" in Example 1) and the serial dilutions of each candidate antibody to be tested (0.001 nM, 0.01 nM, 0.1 nM, 1 nM, 3 nM, 10 nM, and 100 nM) were added, and the plate was incubated at 37 °C for 1 h and then washed. Next, a dilution of His-Tag monoclonal antibody (purchased from Proteintech, Cat. No.: 66005-1-Ig) was added, and the plate was incubated at 37 °C for 1 h and further washed. Subsequently, a dilution of HRP-labeled goat anti-mouse IgG antibody (HRP-conjugated Affinipure Goat Anti-Mouse IgG (H+L), purchased from Proteintech, Cat. No.: SA00001-1) was added, and the plate was incubated at room temperature for 45 min. After the plate was washed, 100 μL of TMB (purchased from Tiangen Biotech, Cat. No.: PA107-01) was added to each well for color development at 37 °C for 15 min, followed by the addition of 50 μL of stopping solution. The absorbance at 450 nm (OD450) was measured using a microplate reader.

The signals of some of the antibodies binding to VEGFA as a function of antibody concentration are shown in FIG. 2.

The VEGFA binding data from the ELISA were fitted to obtain the EC50 values of the antibodies for binding to VEGFA, as shown in Table 7, indicating that the 10 candidate antibodies in the table all had relatively high VEGFA binding activity.

Table 7. Assay for VEGFA binding activity of candidate antibodies

| Name of antibody | Binding to VEGFA-EC50 (nM) |
|---|---|
| V1 | 0.53 |
| V15 | 0.89 |
| V29 | 0.77 |
| V30 | 0.60 |
| V31 | 0.67 |
| V33 | 0.64 |
| V36 | 0.36 |
| V40 | 0.84 |
| V41 | 0.57 |
| V43 | 0.53 |

The amino acid residues in the amino acid sequences of the CDRs of the antibodies described above showed some differences, and the antibody activity fluctuated within an acceptable range.

In addition, the surface plasmon resonance (SPR) technology was utilized to detect the affinity of the nanobodies for VEGFA. Using the Biacore 8K instrument (Cytiva), the antigen VEGFA165 (label-free, purchased from Sino Biological, Cat. No.: HPLC-10008-HNAH) as the ligand was coupled onto a CM5 chip through amino-coupling reagents (EDC and NHS), with the coupling level being about 400 RU. The mobile phase buffer was HBS-EP (0.01 M HEPES pH 7.4, 0.15 M NaCl, 3 mM EDTA, 0.005% v/v Tween 20) during the experiment. The analytes were each candidate antibody and the positive control antibody BI-VEGF ab with the dilution concentrations of 80 nM, 40 nM, 20 nM, 10 nM, 5 nM, and 2.5 nM. In the kinetic analysis, the binding time between the antigen and the antibody was 240 s, the dissociation time was 1500 s, and the flow rate was 30 μL/min. In a multi-cycle mode, the chip was regenerated with 100 mM hydrochloric acid. The binding and dissociation curves of the candidate antibodies with VEGFA165 were fitted using the "1:1 binding" model to obtain the binding rate (ka), dissociation rate (kd), and equilibrium dissociation constant (KD), as shown in Table 8.

Table 8. Binding rate (ka), dissociation rate (kd), and equilibrium dissociation constant (KD) of candidate antibodies with VEGFA165

| Ligand | Analyte | ka (1/Ms) | kd (1/s) | KD (M) |
|---|---|---|---|---|
| VEGFA165 | V1 | 4.09E+05 | 1.01E-04 | 2.47E-10 |
| VEGFA165 | V13 | 3.86E+05 | 8.65E-05 | 2.24E-10 |
| VEGFA165 | V15 | 3.47E+05 | 9.61E-05 | 2.77E-10 |
| VEGFA165 | V29 | 2.91E+05 | 1.27E-04 | 4.36E-10 |
| VEGFA165 | V30 | 6.35E+05 | 1.36E-04 | 2.14E-10 |
| VEGFA165 | V31 | 2.77E+05 | 1.72E-04 | 6.20E-10 |
| VEGFA165 | V33 | 3.23E+05 | 1.68E-04 | 5.22E-10 |
| VEGFA165 | V36 | 4.19E+05 | 1.20E-04 | 2.85E-10 |
| VEGFA165 | V40 | 3.53E+05 | 1.08E-04 | 3.05E-10 |
| VEGFA165 | V41 | 3.31E+05 | 1.60E-04 | 4.82E-10 |
| VEGFA165 | V43 | 4.18E+05 | 8.09E-05 | 1.93E-10 |
| VEGFA165 | BI-VEGF ab | 4.00E+05 | 1.24E-04 | 3.10E-10 |

As can be seen from Table 8, the candidate antibodies had a high affinity for VEGFA165, with KD values differing from the positive control by less than 3-fold, thus it is considered that the affinity of the candidate antibodies was comparable to that of the positive control protein.

## 2. Assay for activity in competing with VEGFR2

To further validate the activity of the candidate antibodies in blocking the binding of VEGFA to its receptor VEGFR2, a competitive ELISA was conducted.

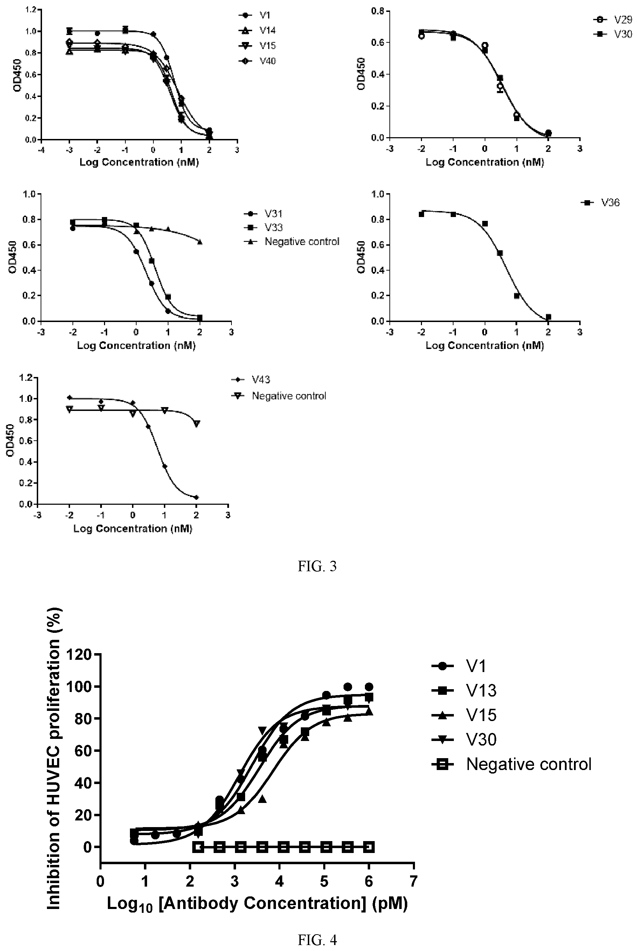

Antigen VEGFA was diluted with ELISA coating buffer (final concentration of 0.53 μg/mL) and added to a 96-well microplate at 100 μL/well for coating at 4 °C overnight. After the plate was blocked with 5% skim milk powder, a VEGFR2 extracellular domain VEGFR2-ECD-Fc (purchased from Sino Biological, Cat. No.: 10012-H02H) at a final concentration of 1 nM was mixed evenly with the serial dilutions of each antibody to be tested (0.01 nM, 0.1 nM, 1 nM, 3 nM, 10 nM, and 100 nM), and the resulting mixture was added to the 96-well plate. The plate was incubated at 37 °C for 1 h and then washed. Subsequently, a dilution of HRP-labeled goat anti-human IgG antibody (HRP-conjugated Goat Anti-Human IgG, purchased from Abbkine, Cat. No.: A21050) was added, and the plate was further incubated at room temperature for 45 min. After the plate was washed, 100 μL of TMB (purchased from Tiangen Biotech, Cat. No.: PA107-01) was added to each well for color development at 37 °C for 15 min, followed by the addition of 50 μL of stopping solution. The absorbance at 450 nm (OD450) was measured using a microplate reader. The competitive ELISA results are shown in FIG. 3.

The comparison of activity of the antibodies in competing with VEGFR2 (IC50 values and maximum inhibition rates) is shown in Table 9, showing that the 12 candidate antibodies in the table could compete with VEGFR2 for binding to VEGFA. The formula for calculating the maximum inhibition rate is as follows:

$$(1 - \frac{\text{OD450 at a candidate antibody concentration of 100 nM}}{\text{OD450 at a candidate antibody concentration of 0.01 nM}}) \times 100\%$$

Table 9. Activity of candidate antibodies in competing with VEGFR2 (IC50 values and maximum inhibition rates)

| Name of antibody | Competing with VEGFR2-IC50 (nM) | Maximum inhibition rate (%) |
|---|---|---|
| V1 | 5.59 | 90.5 |
| V14 | 4.58 | 94.9 |
| V15 | 3.89 | 94.6 |
| V17 | 16.33 | 80.4 |
| V21 | 4.62 | 88.3 |
| V29 | 2.98 | 95.3 |
| V30 | 3.54 | 96.5 |
| V31 | 2.07 | 97.5 |
| V33 | 3.91 | 96.3 |
| V36 | 4.72 | 96.3 |
| V40 | 7.48 | 92.2 |
| V43 | 6.00 | 93.7 |

## 3. Activity of candidate antibodies in inhibiting HUVEC proliferation

The activity of candidate antibodies V1, V13, V15, and V30 in blocking VEGFA-stimulated proliferation of human umbilical vein endothelial cells (HUVECs) was validated.

Primary HUVECs (from the China Center for Type Culture Collection (CCTCC)) were digested, diluted to a concentration of $5 \times 10^4$ cells/mL, and seeded into a 96-well plate at 100 μL/well. The medium was a complete medium (F-12K + 0.1 mg/mL heparin + ECGS + 10% FBS). The cells were allowed for adherent culture for 5 h before the drug treatment was performed.

Mixtures of VEGFA and candidate antibody dilutions were prepared using DMEM/F12 (HAM) (1:1) medium (serum-free, supplemented with penicillin and streptomycin, containing glutamine, purchased from Biological Industries, Cat. No.: 01-172-1ACS). The final concentration of VEGFA in each dilution was 35 ng/mL, and the concentration gradient for the candidate antibody V1 was 1000000 pM, 33333.33 pM, 111111.11 pM, 37037.04 pM, 12345.68 pM, 4115.23 pM, 1371.74 pM, 457.25 pM, 152.42 pM, 50.81 pM, 16.94 pM, and 5.65 pM. The concentration gradient for V13, V15, and V30 was 1000000 pM, 33333.33 pM, 111111.11 pM, 37037.04 pM, 12345.68 pM, 4115.23 pM, 1371.74 pM, 457.25 pM, 152.42 pM, and 5.65 pM.

The negative control antibody was a nanobody binding to an irrelevant antigen, with a concentration gradient of 3-fold serial dilution: 1000000 pM, 33333.33 pM, 111111.11 pM, 37037.04 pM, 12345.68 pM, 4115.23 pM, 1371.74 pM, 457.25 pM, and 152.42 pM.

Meanwhile, control groups were set: one containing no VEGFA (minimal cell proliferation) and another containing VEGFA only without any antibody (maximum cell proliferation). The mixture of VEGFA and each antibody dilution was incubated at 37 °C for 1 h, and after the original medium in the 96-well cell plate was pipetted, the mixture of VEGFA and each antibody dilution was added to the cell plate. Two cell wells were set for each concentration. The cells were cultured in a 37 °C incubator for 72 h. A CCK-8 solution (purchased from Solarbio, Cat. No.: CA1210) was added to the 96-well plate, and the culture plate was incubated in the incubator for 3 h. The absorbance at 450 nm (OD450) was measured using a microplate reader.

The inhibition rates of cell proliferation by the candidate antibodies at different concentrations were calculated based on the difference in readings from the control wells (containing no VEGFA and containing VEGFA only without any antibody). The formula for calculating the inhibition rate is as follows:

$$\frac{[\text{OD450 (}_{\text{containing only VEGFA}}) - \text{OD450 (containing the mixture of VEGFA and candidate antibody dilution)}]}{[\text{OD450 (}_{\text{containing only VEGFA}}) - \text{OD450 (}_{\text{containing no VEGFA}})]} *100\%$$

The inhibition rate curves of the antibodies for HUVEC proliferation, as well as the maximum inhibition rates and IC50 values, are shown in FIG. 4 and Table 10.

Table 10. Maximum inhibition rates and IC50 values of antibodies for HUVEC proliferation

| Candidate antibody | V1 | V13 | V15 | V30 | Negative control |
|---|---|---|---|---|---|
| IC50 (pM) | 2693 | 3939 | 7119 | 1270 | / |
| Maximum inhibition rate (%) | 103.4 | 94.7 | 84.2 | 87.4 | 0 |

Therefore, the four candidate antibodies were all able to inhibit the VEGFA signaling pathway at the cellular level, i.e., inhibit VEGFA-stimulated HUVEC proliferation. At the highest antibody concentration (1 $\mu$M), the candidate antibody V1 was able to efficiently inhibit HUVEC proliferation (with a maximum inhibition rate close to 100%). Judged from IC50, the candidate antibody V30 showed relatively stronger activity.

Through ELISAs for binding to VEGFA and competing with VEGFR2, as well as activity assays for inhibiting HUVEC proliferation at the cellular level, it was demonstrated that the candidate antibodies could efficiently bind to VEGFA and block its binding to VEGFR2, thereby inhibiting the VEGFA-VEGFR signaling pathway and vascular endothelial cell proliferation.

### 4. Assay for binding activity of candidate antibodies to human VEGFA121 and murine VEGFA164

ELISA experiments were conducted using the same method as described in "1. Assay for binding activity of candidate antibodies to VEGFA" in this example, that is, coating with 0.3 $\mu$g/mL human VEGFA121 (purchased from Sino Biological, Cat. No.: 10008-HNAH), or mouse VEGFA164 (purchased from Sino Biological, Cat. No.: 50159-MNAB), or rat VEGFA164 (purchased from Sino Biological, Cat. No.: 80006-RNAB) to detect the binding activity of the His-tagged candidate antibody V1 to human VEGFA121, mouse VEGFA164, and rat VEGFA164.

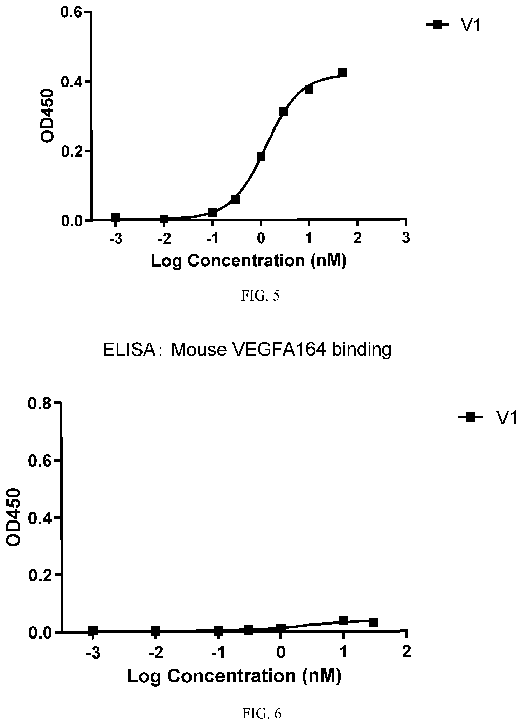

The binding activity of the antibody V1 to human VEGFA121 was detected by ELISA, as shown in FIG. 5 and Table 11.

Table 11. Binding activity of antibody V1 to human VEGFA121

| Antibody | EC50 (nM) for binding to human VEGFA121 |
|---|---|
| V1 | 1.27 |

The binding activity of the antibody V1 to mouse VEGFA164 is shown in FIG. 6.

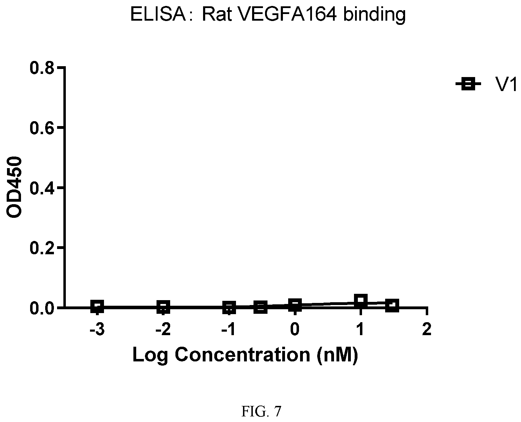

The binding activity of the antibody V1 to rat VEGFA164 is shown in FIG. 7.

In conclusion, the candidate antibody V1 had a higher binding activity to human VEGFA121.

## 5. Detection of *Tm* values of candidate antibodies

Eight candidate antibodies (V1, V15, V29, V30, V31, V36, V40, and V43) were selected, and the buffer in which the antibodies were stored was replaced with: 10 mM His, pH = 6.5, 40 mM NaCl, 5% sucrose, 0.01% Tween-20. The melting temperatures (*Tm*) of the antibodies were measured using the UNCLE instrument (Unchained Labs), and the results are shown in Table 12. The results indicate that these candidate antibodies all possess a certain degree of thermal stability.

Table 12. Melting temperatures (Tm) of candidate antibodies

| Name of antibody | Tm (°C) |
|---|---|
| V1 | 72.6 |
| V15 | 68.7 |
| V29 | 65.3 |
| V30 | 72.8 |
| V31 | 65.6 |
| V36 | 64.6 |
| V40 | 74.3 |
| V43 | 62.4 |

## Example 3: Humanization Engineering of Anti-VEGFA Nanobodies

The candidate antibody V1 was used as the starting antibody for humanization engineering. The sequence of the candidate antibody V1 was first compared with human antibody sequences for homology using the antibody databases IGBLAST and IMGT, and subsequently, the human antibody framework regions with high homology were combined with the CDRs of the candidate antibody to construct complete humanized antibodies. The methods for constructing the expression plasmids of the humanized antibodies and for protein purification were the same as those described in "6. Expression and purification of candidate antibodies" in Example 1.

The purified antibodies were detected for their binding activity to VEGFA using the ELISA method. After the initial humanization engineering of V1, the binding activity of the antibody to VEGFA was significantly decreased. A few amino acids in the framework region were subjected to reverted mutations, resulting in 9 optimized V1 humanized antibodies, whose amino acid sequences are shown in Table 13.

Table 13. Amino acid sequences of humanized candidate antibodies

| SEQ ID NO: x | Name of humanized antibody | Amino acid sequence |
|---|---|---|
| 35 | V1-com-78 | EVQLVESGGGLVQPGGSLRLSCAASGRTFSSYTMGWFRQAPG KEREFVVAISKGGYKYDSVSLEARFTISRDNSKNTVYLQMNSL RAEDTAVYYCAATRAYGSSRLRLADTYEYWGQGTLVTVSS |
| 36 | V1-com-87 | EVQLVESGGGLVQPGGSLRLSCAASGRTFSSYTMGWFRQAPG KEREFVVAISKGGYKYDSVSLEARFTISRDNSKNTLYLQMNSL RPEDTAVYYCAATRAYGSSRLRLADTYEYWGQGTLVTVSS |
| 37 | V1-2m | EVQLVESGGGLVQPGGSLRLSCAASGRTFSSYTMGWFRQAPG KEREFVVAISKGGYKYDSVSLEARFTISRDNSKNTVYLQMNSL RPEDTAVYYCAATRAYGSSRLRLADTYEYWGQGTLVTVSS |

(continued)

| SEQ ID NO: x | Name of humanized antibody | Amino acid sequence |
|---|---|---|
| 38 | V1-3m | EVQLVESGGGLVQPGGSLRLSCAASGRTFSSYTMGWFRQAPGKEREFVVAISKGGYKYDSVSLEARFTISRDNAKNTVYLQMNSLRPEDTAVYYCAATRAYGSSRLRLADTYEYWGQGTLVTVSS |
| 39 | V1-com-74-78-97 | EVQLVESGGGLVQPGGSLRLSCAASGRTFSSYTMGWFRQAPGKEREFVVAISKGGYKYDSVSLEARFTISRDNAKNTVYLQMNSLRAEDTAVYYCASTRAYGSSRLRLADTYEYWGQGTLVTVSS |
| 40 | V1-4m | EVQLVESGGGLVQPGGSLRLSCAASGRTFSSYTMGWFRQAPGKEREFVVAISKGGYKYDSVSLEARFTISRDNAKNTVYLQMNSLRPEDTAVYYCASTRAYGSSRLRLADTYEYWGQGTLVTVSS |
| 41 | V1-SA1 | EVQLVESGGGLVQPGGSLRLSCAVSGRTFSSYTMGWFRQAPGKEREFVVAISKGGYKYDSVSLEARFTISRDNAKNTVYLQMNSLRPEDTAVYYCAATRAYGSSRLRLADTYEYWGQGTLVTVSS |
| 42 | V1-SA3 | EVQLVESGGGLVQPGGSLRLSCAASGRTFSSYTMGWFRQAPGKEREFVVAISKGGYKYDSVSLEARFTISRDNAKNTVYLQMNSLRAEDTAVYYCAATRAYGSSRLRLADTYEYWGQGTLVTVSS |
| 43 | V1-DP | EVQLVESGGGLVQPGGSLRLSCAVSGRTFSSYTMGWFRQAPGKEREFVVAISKGGYKYDSVSLEARFTISRDNSKNTVYLQMNSLRPEDTAVYYCAATRAYGSSRLRLADTYEYWGQGTLVTVSS |
| 44 | V13-2m | EVQLVESGGGLVQPGGSLRLSCAASGRTFTSYTMGWFRQAPGKEREFVVAISKGGYKYDAVSLEARFTISRDNSKNTVYLQMNSLRPEDTAVYYCAGTRAYGSSRLRLADTYEYWGQGTLVTVSS |
| 45 | V13-4m | EVQLVESGGGLVQPGGSLRLSCAASGRTFTSYTMGWFRQAPGKEREFVVAISKGGYKYDAVSLEARFTISRDNAKNTVYLQMNSLRPEDTAVYYCAGTRAYGSSRLRLADTYEYWGQGTLVTVSS |
| 46 | V15-2m | EVQLVESGGGLVQPGGSLRLSCAASGRTFASYTMGWFRQAPGKEREFVVAISKGGYKYDSVSLEARFTISRDNSKNTVYLQMNSLRPEDTAVYYCAGTRAYGSSRLRLAETYEYWGQGTLVTVSS |
| 47 | V15-4m | EVQLVESGGGLVQPGGSLRLSCAASGRTFASYTMGWFRQAPGKEREFVVAISKGGYKYDSVSLEARFTISRDNAKNTVYLQMNSLRPEDTAVYYCAGTRAYGSSRLRLAETYEYWGQGTLVTVSS |
| 48 | V30-2H-78 | EVQLVESGGGLVQPGGSLRLSCAASGRTFSSYTMGWFRQAPGKEREFVVAISKGGYKYDAVSLEARFTISRDNSKNTVYLQMNSLRAEDTAVYYCAATRAYGSSRLRLAKTYEYWGQGTLVTVSS |
| 49 | V30-2H-87 | EVQLVESGGGLVQPGGSLRLSCAASGRTFSSYTMGWFRQAPGKEREFVVAISKGGYKYDAVSLEARFTISRDNSKNTLYLQMNSLRPEDTAVYYCAATRAYGSSRLRLAKTYEYWGQGTLVTVSS |

(continued)

| SEQ ID NO: x | Name of humanized antibody | Amino acid sequence |
|---|---|---|
| 50 | V30-2H-2m | EVQLVESGGGLVQPGGSLRLSCAASGRTFSSYTMGWFRQAPGKEREFVVAISKGGYKYDAVSLEARFTISRDNSKNTVYLQMNSLRPEDTAVYYCAATRAYGSSRLRLAKTYEYWGQGTLVTVSS |
| 51 | V30-3H-2m | EVQLVESGGGLVQPGGSLRLSCAASGRTFSSYTMGWFRQAPGKGREFVVAISKGGYKYDAVSLEARFTISRDNSKNTVYLQMNSLRPEDTAVYYCAATRAYGSSRLRLAKTYEYWGQGTLVTVSS |
| 52 | V40-3H-2m | EVQLVESGGGLVQPGGSLRLSCAASGRTFSSYTMGWFRQAPGKEREFVVAISKGGYKYNAVSLEARFTISRDNSKNTVYLQMNSLRPEDTAVYYCAATRAYGSSRLRLAKTYEYWGQGTLVTVSS |
| 53 | V40-3H-4m | EVQLVESGGGLVQPGGSLRLSCAASGRTFSSYTMGWFRQAPGKEREFVVAISKGGYKYNAVSLEARFTISRDNAKNTVYLQMNSLRPEDTAVYYCASTRAYGSSRLRLAKTYEYWGQGTLVTVSS |
| 54 | V40-4H-2m | EVQLVESGGGLVQPGGSLRLSCAASGRTFTSYTMGWFRQAPGKEREFVVAISKGGYKYNAVSLEARFTISRDNSKNTVYLQMNSLRPEDTAVYYCAATRAYGSSRLRLAKTYEYWGQGTLVTVSS |
| 55 | V40-4H-4m | EVQLVESGGGLVQPGGSLRLSCAASGRTFTSYTMGWFRQAPGKEREFVVAISKGGYKYNAVSLEARFTISRDNAKNTVYLQMNSLRPEDTAVYYCASTRAYGSSRLRLAKTYEYWGQGTLVTVSS |
| 56 | V43-1H-78 | EVQLVESGGGLVQPGGSLRLSCAASGRTFSSYTMGWFRQAPGKEREFVVAISKGGYKYDSVSLEARFTISRDNSKNTVYLQMNSLRAEDTAVYYCAATRAYGSSRLRLANTYEYWGQGTLVTVSS |
| 57 | V43-1H-87 | EVQLVESGGGLVQPGGSLRLSCAASGRTFSSYTMGWFRQAPGKEREFVVAISKGGYKYDSVSLEARFTISRDNSKNTLYLQMNSLRPEDTAVYYCAATRAYGSSRLRLANTYEYWGQGTLVTVSS |
| 58 | V43-1H-2m | EVQLVESGGGLVQPGGSLRLSCAASGRTFSSYTMGWFRQAPGKEREFVVAISKGGYKYDSVSLEARFTISRDNSKNTVYLQMNSLRPEDTAVYYCAATRAYGSSRLRLANTYEYWGQGTLVTVSS |
| 59 | V43-3H-78 | EVQLVESGGGLVQPGGSLRLSCAASGRTFTSYTMGWFRQAPGKEREFVVAISKGGYKYDSVSLEARFTISRDNSKNTVYLQMNSLRAEDTAVYYCAGTRAYGSSRLRLANTYEYWGQGTLVTVSS |
| 60 | V43-3H-87 | EVQLVESGGGLVQPGGSLRLSCAASGRTFTSYTMGWFRQAPGKEREFVVAISKGGYKYDSVSLEARFTISRDNSKNTLYLQMNSLRPEDTAVYYCAGTRAYGSSRLRLANTYEYWGQGTLVTVSS |
| 61 | V43-4m | EVQLVESGGGLVQPGGSLRLSCAASGRTFTSYTMGWFRQAPGKEREFVVAISKGGYKYDSVSLEARFTISRDNAKNTVYLQMNSLRPEDTAVYYCAGTRAYGSSRLRLANTYEYWGQGTLVTVSS |

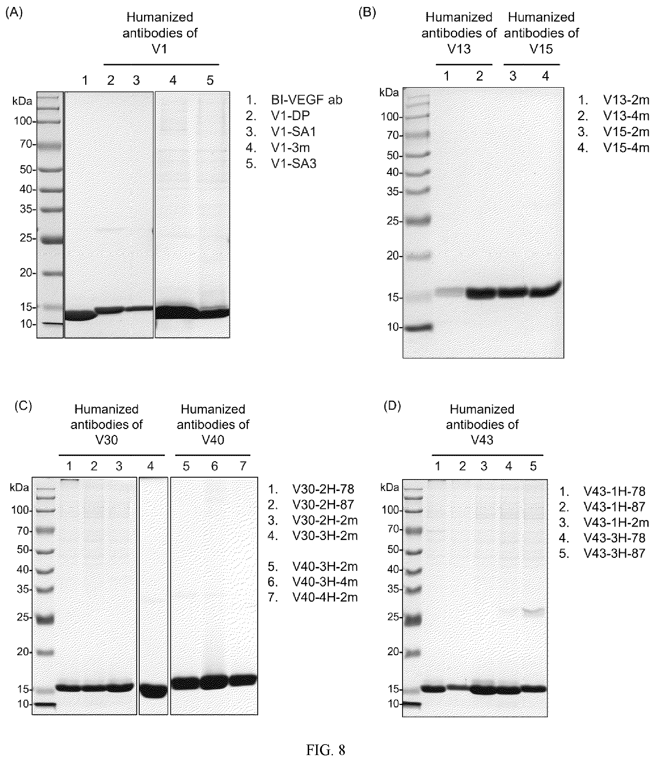

The humanized antibodies with reverted mutations were subjected to transient expression and purification, and the experimental procedure for the first step of purification was the same as that described in "6. Expression and purification of candidate antibodies" in Example 1. The positive control BI-VEGF ab and humanized antibodies V1-DP and V1-SA1, after Ni-bead affinity purification, underwent cation exchange chromatography purification (S column). The SDS-PAGE Coomassie-stained gel images of some of the proteins after purification are shown in FIG. 8(A), and the target protein molecular weight was in line with the expected value (about 15 kDa).

The CDRs of candidate antibodies V13, V15, V30, V40, and V43 were grafted onto the framework regions of the V1 humanized antibodies with reverted mutations, and the humanization engineering of these candidate antibodies was carried out, followed by expression and purification. Some of the humanized antibodies after purification are shown in FIGs. 8(B), 8(C), and 8(D), and the target protein molecular weight was in line with the expected value (about 15 kDa).

### Example 4: Activity Assay for Humanized Anti-VEGFA Nanobodies

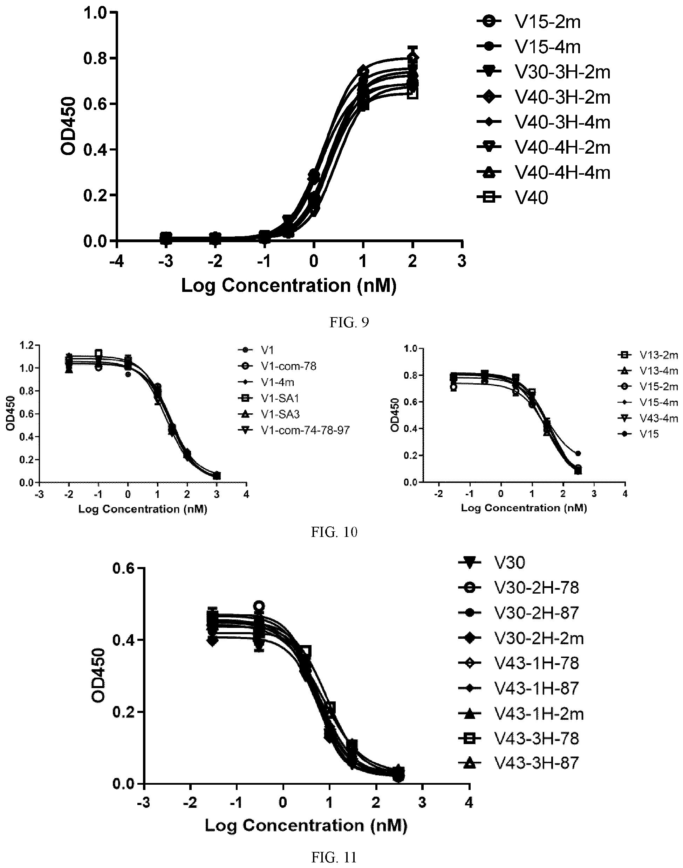

The humanized antibodies obtained were subjected to VEGFA binding activity assay using the ELISA method, and the experimental procedure was the same as that described in "1. Assay for binding activity of candidate antibodies to VEGFA" in Example 2. The ELISA results for the binding of some of the humanized antibodies to VEGFA are shown in FIG. 9.

The VEGFA binding data from the ELISA were fitted to obtain the EC50 values of the humanized antibodies for binding to VEGFA, as shown in Table 14. The data indicate that the binding activity of the candidate antibodies to VEGFA after humanization engineering was similar to that before humanization (V40).

Table 14. EC50 values of humanized antibodies for binding to VEGFA

| Name of antibody | Binding to VEGFA-EC50 (nM) |
| --- | --- |
| V15-2m | 2.04 |
| V15-4m | 1.42 |
| V30-3H-2m | 1.38 |
| V40-3H-2m | 1.64 |
| V40-3H-4m | 2.05 |
| V40-4H-2m | 2.82 |
| V40-4H-4m | 2.34 |
| V40 | 2.03 |

To further validate the activity of the humanized antibodies in blocking the binding of VEGFA to its receptor VEGFR2, a competitive ELISA was conducted. The experimental procedure was similar to that described in "2. Assay for activity in competing with VEGFR2" in Example 2, with the difference being that for the humanized antibodies of V1, V13, V15, and V43, the final concentration of the VEGFR2 extracellular domain VEGFR2-ECD-Fc (purchased from Sino Biological, Cat. No.: 10012-H02H) was 2 nM. The concentration gradient for the humanized antibodies of V1 was: 0.01 nM, 0.1 nM, 1 nM, 10 nM, 30 nM, 100 nM, and 1000 nM. The concentration gradient for the V13, V15, and V43 humanized antibodies was: 0.03 nM, 0.3 nM, 3 nM, 10 nM, 30 nM, and 300 nM.

The results show that the activity of the humanized antibodies of V1 and V15 in competing with VEGFR2 for binding to VEGFA was restored to their pre-humanization levels (i.e., V1 and V15), while the remaining humanized antibodies exhibited similar activity in competing with VEGFR2. The competitive ELISA results for some of the humanized antibodies are shown in FIG. 10, and the IC50 values and maximum inhibition rates are shown in Table 15. The formula for calculating the maximum inhibition rate is as follows:

$$(1 - \frac{\text{OD450 at the highest antibody concentration}}{\text{OD450 at the lowest antibody concentration}}) \times 100\%$$

Table 15. Competitive ELISA results and maximum inhibition rates for humanized antibodies

| Name of antibody | Competing with VEGFR2-IC50 (nM) | Maximum inhibition rate (%) |
| --- | --- | --- |
| V1 | 26.45 | 95.0 |

(continued)

| Name of antibody | Competing with VEGFR2-IC50 (nM) | Maximum inhibition rate (%) |
|---|---|---|
| V1-com-78 | 28.51 | 93.7 |
| V1-4m | 26.13 | 93.0 |
| V1-SA1 | 22.48 | 94.6 |
| V1-SA3 | 26.68 | 93.5 |
| V1-com-74-78-97 | 20.04 | 94.4 |
| V13-2m | 40.14 | 88.9 |
| V13-4m | 26.8 | 89.2 |
| V15-2m | 31.68 | 84.8 |
| V15-4m | 25.96 | 87.2 |
| V43-4m | 35.45 | 89.3 |
| V15 | 29.34 | 71.6 |

In addition, ELISA for blocking VEGFR2 was conducted on the humanized antibodies of V30 and the remaining humanized antibodies of V43. The experimental procedure was similar to that described in "2. Assay for activity in competing with VEGFR2" in Example 2, with the difference being that the final concentration of the VEGFR2 extracellular domain VEGFR2-ECD-Fc (purchased from Sino Biological, Cat. No.: 10012-H02H) was 1 nM. The concentration gradient for the humanized antibodies was: 0.03 nM, 0.3 nM, 3 nM, 10 nM, 30 nM, and 300 nM.

The ELISA results for the humanized antibodies of V30 and V43 competing with VEGFR2 are shown in FIG. 11.

The activity of the antibodies in competing with VEGFR2 (IC50 values and maximum inhibition rates) is shown in Table 16, showing that the humanized antibodies could compete with VEGFR2 for binding to VEGFA, with the activity similar to that before humanization (V30). The formula for calculating the maximum inhibition rate is as follows:

$$(1 - \frac{\text{OD450 at an antibody concentration of 300 nM}}{\text{OD450 at an antibody concentration of 0.03 nM}}) \times 100\%$$

Table 16. Activity of antibodies in competing with VEGFR2 (IC50 values and maximum inhibition rates)

| Name of antibody | Competing with VEGFR2-IC50 (nM) | Maximum inhibition rate (%) |
|---|---|---|
| V30 | 5.72 | 94.8 |
| V30-2H-78 | 4.97 | 95.5 |
| V30-2H-87 | 5.81 | 94.2 |
| V30-2H-2m | 5.64 | 94.7 |
| V43-1H-78 | 5.62 | 95.5 |
| V43-1H-87 | 5.27 | 95.4 |
| V43-1H-2m | 5.99 | 94.5 |
| V43-3H-78 | 8.63 | 93.4 |
| V43-3H-87 | 7.15 | 91.6 |

The surface plasmon resonance (SPR) technology was utilized to couple the antigen VEGFA165, so as to detect the affinity of the nanobodies for VEGFA before and after humanization engineering. The experimental procedure and the data fitting method were the same as those described in "1. Assay for binding activity of candidate antibodies to VEGFA" in Example 2. The obtained KD values are shown in Table 17.

Table 17. Binding rate (ka), dissociation rate (kd), and equilibrium dissociation constant (KD) of humanized antibodies with VEGFA

| Ligand | Analyte | ka (1/Ms) | kd (1/s) | KD (M) |
|---|---|---|---|---|
| VEGFA165 | V1 | 4.09E+05 | 1.01E-04 | 2.47E-10 |
| VEGFA165 | V1-2m | 3.39E+05 | 8.24E-05 | 2.43E-10 |
| VEGFA165 | V1-com-78 | 3.47E+05 | 8.91E-05 | 2.57E-10 |
| VEGFA165 | V1-com-87 | 3.60E+05 | 9.08E-05 | 2.52E-10 |

In addition, the surface plasmon resonance (SPR) technology was utilized to detect the affinity of the humanized antibody V1-SA1 for VEGFA in a manner of coupling the antibody. Using the Biacore 8K instrument (Cytiva), the antibody V1-SA1 as the ligand was coupled onto a CM5 chip through amino-coupling reagents (EDC and NHS), with the coupling conditions being 10 mM sodium acetate (pH 4.0) and a V1-SA1 protein concentration of 20 μg/mL. The coupling level of the ligand V1-SA1 was 371.8 RU. The mobile phase buffer was HBS-EP (0.01 M HEPES pH 7.4, 0.15 M NaCl, 3 mM EDTA, 0.005% v/v Tween 20) during the experiment. The analyte was the antigen VEGFA165 (label-free, purchased from GenScript, Cat. No.: Z03073) with the dilution concentrations of 100 nM, 50 nM, 25 nM, 12.5 nM, 6.25 nM, 3.125 nM, and 1.5625 nM. In the kinetic analysis, the binding time between the antigen and the antibody was 180 s, the dissociation time was 200 s, and the flow rate was 30 μL/min. In a multi-cycle mode, the chip was regenerated with 10 mM glycine hydrochloride (pH = 1.5). The antigen-antibody binding curve was fitted by adopting the "two state reaction" model to obtain the binding rate, the dissociation rate, and the KD.

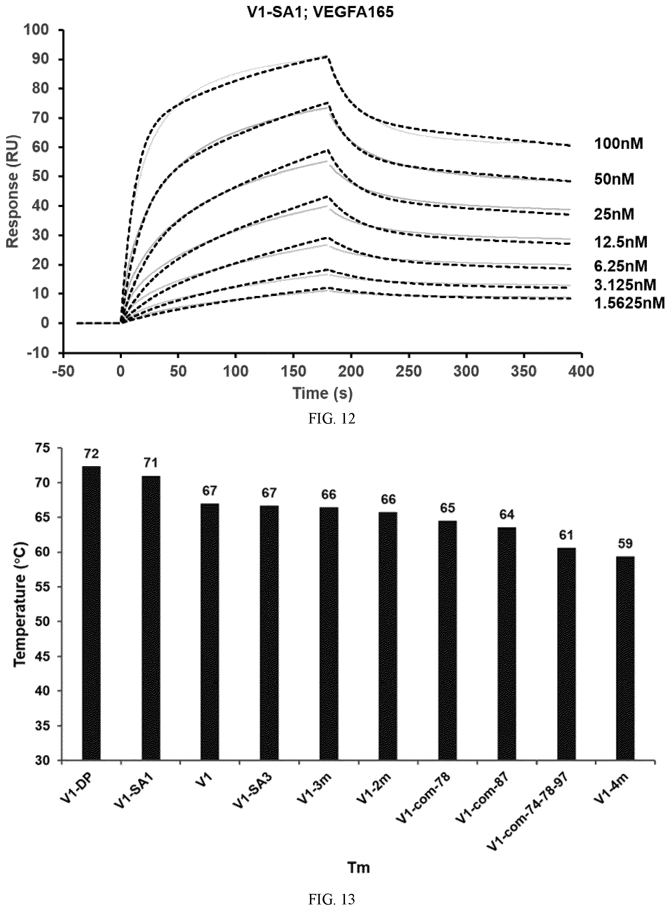

The binding and dissociation curves of V1-SA1 with VEGFA165 are shown in FIG. 12, and the fitted KD is shown in Table 18.

Table 18. Binding rate (ka), dissociation rate (kd), and equilibrium dissociation constant (KD) of humanized antibody V1-SA1 with VEGFA

| Ligand | Analyte | ka1 (1/Ms) | kd1 (1/s) | ka2 (1/s) | kd2 (1/s) | KD (M) |
|---|---|---|---|---|---|---|
| V1-SA1 | VEGFA165 | 1.06E+06 | 5.98E-02 | 9.41E-03 | 7.95E-04 | 4.38E-09 |

It can be seen from Tables 17 and 18 that the antibody V1 maintained a high affinity for VEGFA165 after humanization engineering.

## Example 5: Stability Study of Humanized Anti-VEGFA Nanobodies

### 1. Comparison of *Tm* values of humanized antibodies of V1

Antibodies with high thermal stability are more favorable for the production and long-term storage of antibodies. Therefore, the melting temperatures (*Tm*) of different humanized antibodies were compared.

The buffer for 9 humanized antibodies of V1 was replaced with PBS (pH = 7.4), and the *Tm* was measured using the UNCLE instrument (Unchained Labs). The ranking of *Tm* values is shown in FIG. 13. The *Tm* values of the humanized antibodies fluctuated within an acceptable range, with V1-DP and V1-SA1 having higher *Tm* values, indicating that the thermal stability of these two antibodies was likely superior to that of the other humanized antibodies.

### 2. Colloidal stability study of humanized antibody V1-SA1

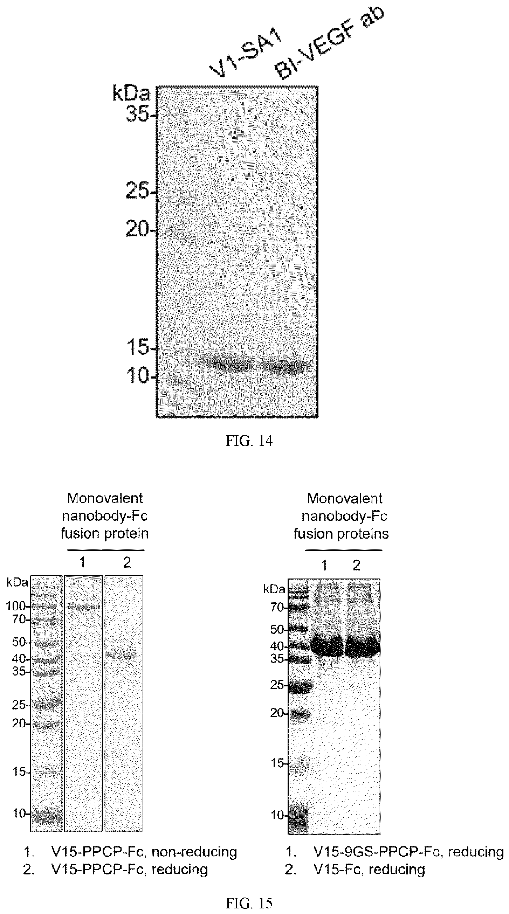

The coding genes of the humanized antibody V1-SA1 and the positive control antibody BI-VEGF ab were each constructed into the expression vector pCDNA3.1(+). A secretion peptide of MDAMKRGLCCVLLLCGAVFVSPS (SEQ ID NO: 68) was added to the N terminus of each antibody, while a flexible linker GGS and a 6×His tag were sequentially added to the C terminus of the antibody. Transient expression and Ni-bead affinity purification were carried out for these two antibodies, and the experimental procedure was the same as that described in "6. Expression and purification of candidate antibodies" in Example 1. After purification, the proteins were subjected to SDS-PAGE separation, and the gel staining results are shown in FIG. 14. It can be seen from the figure that the molecular weights of the two proteins were in line with the expected value (about 15 kDa). The protein expression levels and the purity in SDS-PAGE Coomassie-stained gel are shown in Table 19, indicating that the expression level of V1-SA was much higher than that of BI-VEGF ab.

Table 19. Transient expression levels and protein purity in SDS-PAGE gel of humanized antibody V1-SA1 and positive control antibody BI-VEGF ab

| Name of protein | Expression level/L | Protein purity in SDS-PAGE Coomassie-stained gel |
|---|---|---|
| V1-SA1 | 37.3 mg | 100% |
| BI-VEGF ab | 13.3 mg | 99.7% |

Both V1-SA1 and BI-VEGF ab proteins were buffer-exchanged into $1\times$ PBS (pH = 7.4), with the protein concentration adjusted to 10 mg/mL. HPLC-SEC analysis (chromatography column: Zenix-C SEC-300, Sepax) was performed on the proteins. The proportions of protein monomers, aggregates, and fragments are shown in Table 20. It can be seen that the proportion of aggregates of V1-SA was much lower than that of BI-VEGF ab, suggesting that V1-SA1 is less prone to aggregation.

Table 20. Proportion of each component in SEC-HPLC analysis of antibodies V1-SA1 and BI-VEGF ab

| Name of protein | Proportion of high-molecular-weight aggregates (%) | Proportion of monomers (%) | Proportion of protein fragments (%) |
|---|---|---|---|
| V1-SA1 | 0.20 | 98.55 | 1.25 |
| BI-VEGF ab | 3.25 | 95.49 | 1.27 |

Antibodies V1-SA1 and BI-VEGF ab (with a protein concentration of 10 mg/mL) that were buffer-exchanged into PBS were filtered sterility and left to stand at 37 °C for a period of time (0 days and 2 days). The appearance of the protein solutions was observed, and the colloidal stability of the two was compared, with the results shown in Table 21.

Table 21. Comparison of appearance, average particle size, and PDI of antibodies V1-SA1 and BI-VEGF ab left to stand at 37 °C for 0 days and 2 days

| Examination condition | Detection indicator | V1-SA1 | BI-VEGF ab |
|---|---|---|---|
| 37°C for 0 days | Solution appearance | Clear | Clear |
| | Z-Average ($\pm$ SD) (d.nm) | 6.6 $\pm$ 3.9 | 46.7 $\pm$ 44.3 |
| | Polydispersity Index (PDI) | 0.343 | 0.902 |
| 37 °C for 2 days | Solution appearance | Clear | Precipitation observed |
| | Z-Average ($\pm$ SD) (d.nm) | 5.9 $\pm$ 3.7 | 127.3 $\pm$ 62.2 |
| | Polydispersity Index (PDI) | 0.380 | 0.238 |

Based on the principle of dynamic light scattering, a nanoparticle size and potential analyzer (NS-90Z, OMEC) was used to measure the protein particle size of the solutions of the antibodies V1-SA1 and BI-VEGF ab (diluted 4-fold with PBS) left to stand at 37 °C for a period of time (0 day and 2 days) described above. The results of the protein average particle size (Z-average) and polydispersity index (PDI) are shown in Table 21. For the 0-hour samples, the average hydrodynamic diameter and PDI of V1-SA1 were both smaller than those of BI-VEGF ab. Considering that the molecular weights of the two are similar, this indicates that BI-VEGF ab had a certain proportion of high-molecular-weight protein aggregates in this buffer, while under the same conditions, V1-SA1 exhibited a more uniform particle size distribution and a significantly lower proportion of high-molecular-weight protein aggregates as compared to BI-VEGF Ab.

It can be seen from Table 21 that for the samples left to stand at 37 °C for 2 days, V1-SA1 remained clear, while the BI-VEGF ab protein solution showed precipitation, indicating that the colloidal stability of V1-SA1 was better than that of BI-VEGF ab. At this time, the average hydrodynamic diameter of V1-SA1 was much smaller than that of BI-VEGF ab and showed relatively little change compared to the 0-hour sample; whereas the average hydrodynamic diameter of BI-VEGF ab was much larger than that of V1-SA1 and showed a significant increase compared to the 0-hour BI-VEGF ab solution, indicating that BI-VEGF ab further formed high-molecular-weight protein aggregates under high-temperature conditions.

**Example 6: Preparation and Activity Study of Monovalent Nanobody-Fc Fusion Proteins**

## 1. Preparation of monovalent nanobody-Fc fusion proteins

In this example, the fusion expression of a monovalent nanobody with IgG1 Fc (SEQ ID NO: 62) or PPCP-Fc (SEQ ID NO: 75, the remaining amino acid sequence after removal of DKTHTC in SEQ ID NO: 62) or IgG1 Fc mutant Fc-m1 (SEQ ID NO: 63) was carried out.

The amino acid sequence of the IgG1 Fc is as follows:

DKTHTCPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKSRWQQGN
VFSCSVMHEALHNHYTQKSLSLSPGK (SEQ ID NO: 62)

The amino acid sequence of the PPCP-Fc is as follows:

PPCPAPELLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSV
MHEALHNHYTQKSLSLSPGK (SEQ ID NO: 75)

The amino acid sequence of the IgG1 Fc mutant Fc-m1 is as follows:

DKTHTCPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDKSRWQQGN
VFSCSVMHEALHNHYTQKSLSLSPGK (SEQ ID NO: 63)

The amino acid sequences of the constructed nanobody-Fc fusion proteins are shown in Table 22.

Table 22. Amino acid sequences of nanobody-Fc fusion proteins

| SEQ ID NO: x | Monovalent nanobody-Fc fusion protein | Amino acid sequence of monovalent nanobody-Fc fusion protein |
|---|---|---|
| 64 | V15-PPCP-Fc | QLQLVESGGGSVQPGGSLRLSCEVSGRTFASYTMGWFRQAPGK EREFVVAISKGGYKYDSVSLEARFTISKDNTKNTVYLQMNSLKP EDTAVYYCAGTRAYGSSRLRLAETYEYWGQGTQVTVSSPPCPA PELLGGPSVFLFPPKPKDTLMISRTPEVTCVVVDVSHEDPEVKFN WYVDGVEVHNAKTKPREEQYNSTYRVVSVLTVLHQDWLNGK EYKCKVSNKALPAPIEKTISKAKGQPREPQVYTLPPSRDELTKN QVSLTCLVKGFYPSDIAVEWESNGQPENNYKTTPPVLDSDGSFF LYSKLTVDKSRWQQGNVFSCSVMHEALHNHYTQKSLSLSPGK |
| 65 | V15-9GS-PPCP-Fc | QLQLVESGGGSVQPGGSLRLSCEVSGRTFASYTMGWFRQAPGK |

(continued)

| SEQ ID NO: x | Monovalent nanobody-Fc fusion protein | Amino acid sequence of monovalent nanobody-Fc fusion protein |
|---|---|---|
| | | EREFVVAISKGGYKYDSVSLEARFTISKDNTKNTVYLQMNSLKP EDTAVYYCAGTRAYGSSRLRLAETYEYWGQGTQVTVSSGGGG SGGGSPPCPAPELLGGPSVFLFPPKPKDTLMISRTPEVTCVVVDV SHEDPEVKFNWYVDGVEVHNAKTKPREEQYNSTYRVVSVLTV LHQDWLNGKEYKCKVSNKALPAPIEKTISKAKGQPREPQVYTL PPSRDELTKNQVSLTCLVKGFYPSDIAVEWESNGQPENNYKTTP PVLDSDGSFFLYSKLTVDKSRWQQGNVFSCSVMHEALHNHYTQ KSLSLSPGK |
| 66 | V15-Fc | QLQLVESGGGSVQPGGSLRLSCEVSGRTFASYTMGWFRQAPGK EREFVVAISKGGYKYDSVSLEARFTISKDNTKNTVYLQMNSLKP EDTAVYYCAGTRAYGSSRLRLAETYEYWGQGTQVTVSSDKTH TCPPCPAPELLGGPSVFLFPPKPKDTLMISRTPEVTCVVVDVSHE DPEVKFNWYVDGVEVHNAKTKPREEQYNSTYRVVSVLTVLHQ DWLNGKEYKCKVSNKALPAPIEKTISKAKGQPREPQVYTLPPSR DELTKNQVSLTCLVKGFYPSDIAVEWESNGQPENNYKTTPPVLD SDGSFFLYSKLTVDKSRWQQGNVFSCSVMHEALHNHYTQKSLS LSPGK |
| 67 | V1-SA1-Fc-m1 | EVQLVESGGGLVQPGGSLRLSCAVSGRTFSSYTMGWFRQAPGK EREFVVAISKGGYKYDSVSLEARFTISRDNAKNTVYLQMNSLRP EDTAVYYCAATRAYGSSRLRLADTYEYWGQGTLVTVSSEPKSA DKTHTCPPCPAPELLGGPSVFLFPPKPKDTLMISRTPEVTCVVVD VSHEDPEVKFNWYVDGVEVHNAKTKPREEQYNSTYRVVSVLT VLHQDWLNGKEYKCKVSNKALPAPIEKTISKAKGQPREPQVYT LPPSREEMTKNQVSLTCLVKGFYPSDIAVEWESNGQPENNYKTT PPVLDSDGSFFLYSKLTVDKSRWQQGNVFSCSVMHEALHNHYT QKSLSLSPGK |

To facilitate the comparison of the VEGFA binding activity between the linear tandem bivalent nanobody 2V15-9GS (SEQ ID NO: 74) (which contains a 6-histidine tag based on 2V15-9GS) and the fusion proteins V15-PPCP-Fc, V15-9GS-PPCP-Fc, and V15-Fc, a linker (SGGS (SEQ ID NO: 70)) and a 6-histidine tag (HHHHHH (SEQ ID NO: 71)) were added to the C terminus of the Fc segment in the fusion proteins. During the plasmid construction for the monovalent nanobody-Fc fusion proteins, the nanobody and the Fc containing the linker and histidine tag were amplified separately using the PCR method, with the PCR template for the Fc fragment derived from a genetically synthesized Fc gene. A two-fragment homologous recombination method was used to integrate the nanobody and Fc into the vector pCDNA3.1(+), and the enzyme cutting sites used were NheI and XbaI. The secretion peptide of the fusion protein was METDTLLLWVLLLWVPGSTG (SEQ ID NO: 72), followed by the fusion of the target protein sequence. After successful sequencing of these fusion protein plasmids, endotoxin-free plasmid extraction was performed. Polyethylenimine (abbreviated as PEI, linear, MW = 25 kDa, purchased from Polysciences, Cat. No.: 23966-1), a transfection reagent, was used to perform transient transfection on 293F suspension cells. The cell supernatant was collected on day 4 or day 5 after transfection, and Ni-bead purification was carried out. The purified proteins were analyzed by SDS-PAGE, and under reducing conditions, the molecular weight of the protein monomers was about 39 kDa, which was in line with the expected value. The Coomassie-stained gel images of some of the monovalent nanobody-Fc fusion proteins after purification are shown in FIG. 15.

2V15-9GS (SEQ ID NO: 74)
QLQLVESGGGSVQPGGSLRLSCEVSGRTFASYTMGWFRQAPGKEREFVVAISKGGYKYDSVSLEARF
TISKDNTKNTVYLQMNSLKPEDTAVYYCAGTRAYGSSRLRLAETYEYWGQGTQVTVSSGGGGSGG

GSQLQLVESGGGSVQPGGSLRLSCEVSGRTFASYTMGWFRQAPGKEREFVVAISKGGYKYDSVSLEA
RFTISKDNTKNTVYLQMNSLKPEDTAVYYCAGTRAYGSSRLRLAETYEYWGQGTQVTVSS

For the fusion protein (V1-SAI-Fc-m1) of the humanized antibody V1-SA1 with Fc, the amino acid sequence of the linker between V1-SA1 and Fc-m1 is as follows: EPKSA (SEQ ID NO: 73). An expression plasmid was constructed using the method described above, but without additional amino acids at the C terminus of Fc, meaning that the fusion protein V1-SA1-Fc-m1 contained a Fc tag but no histidine tag. After transient transfection of 293F cells with the V1-SA1-Fc-m1 expression plasmid, Protein A bead affinity purification was performed. During protein elution, 0.1 M citric acid (pH 3.0) was used to elute the target protein, followed by rapid neutralization to obtain the target protein.

### 2. Activity assay for monovalent nanobody-Fc fusion proteins

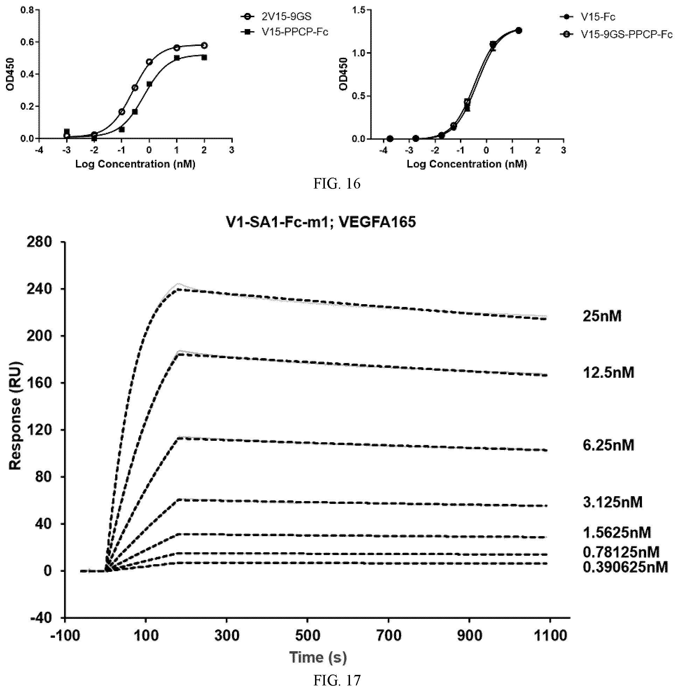

The purified monovalent nanobody-Fc fusion proteins were subjected to VEGFA binding activity assay, and the ELISA experimental procedure was the same as that described in "1. Assay for binding activity of candidate antibodies to VEGFA" in Example 2. The ELISA results are shown in FIG. 16, and the EC50 values are shown in Table 23.

Table 23. VEGFA binding activity assay for monovalent nanobody-Fc fusion proteins

| Monovalent nanobody-Fc fusion protein or tandem bivalent nanobody | EC50 (nM) for binding to VEGFA |
|---|---|
| V15-PPCP-Fc | 0.64 |
| V15-9GS-PPCP-Fc | 0.35 |
| V15-Fc | 0.43 |
| 2V15-9GS | 0.25 |

As can be seen from the data in the table, the tested monovalent nanobody-Fc fusion proteins and linear tandem bivalent nanobodies both had strong VEGFA binding activity.

The surface plasmon resonance (SPR) technology was utilized to detect the affinity of the monovalent nanobody-Fc fusion protein (V1-SA1-Fc-m1) for VEGFA. Using the Biacore 8K instrument (Cytiva), V1-SA1-Fc-m1 as the ligand was captured onto a Protein A chip. During immobilization, V1-SA1-Fc-m1 was diluted to a protein concentration of 2 $\mu$g/mL with the mobile phase buffer HBS-EP (0.01 M HEPES pH 7.4, 0.15 M NaCl, 3 mM EDTA, 0.005% v/v Tween 20), and its coupling level was about 590 RU. During the experiment, the analyte was the antigen VEGFA165 (label-free, purchased from GenScript, Cat. No.: Z03073) with the dilution concentrations of 25 nM, 12.5 nM, 6.25 nM, 3.125 nM, 1.5625 nM, 0.78125 nM, and 0.390625 nM. In the kinetic analysis, the binding time between the fusion protein and the antigen VEGFA165 was 180 s, the dissociation time was 900 s, and the flow rate was 30 $\mu$L/min. After each "binding-dissociation" cycle, the chip was regenerated under the condition of 10 mM glycine hydrochloride (pH 1.5). The binding and dissociation curves of the fusion protein with antigen VEGFA165 are shown in FIG. 17. The "1:1 binding" model was used for fitting to obtain the binding rate (ka), dissociation rate (kd), and equilibrium dissociation constant (KD), as shown in Table 24.

Table 24. Binding rate (ka), dissociation rate (kd), and equilibrium dissociation constant (KD) of monovalent nanobody-Fc fusion protein with VEGFA165

| Ligand | Analyte | ka (1/Ms) | kd (1/s) | KD (M) |
|---|---|---|---|---|
| V1-SA1-Fc-m1 | VEGFA165 | 8.77E+05 | 1.30E-04 | 1.48E-10 |

It can be seen from the SPR data that the monovalent nanobody-Fc fusion protein V1-SA1-Fc-m1 had a high affinity for VEGFA165 (KD = 0.148 nM). In contrast, as shown in Table 18 and FIG. 12 of the "6. Activity assay for humanized antibodies" in Example 2 described above, the KD of monovalent nanobody V1-SA1 with VEGFA165 was 4.38 nM. Therefore, after the monovalent nanobody was fused with Fc, its affinity for VEGFA165 increased by nearly 30-fold compared to the nanobody without Fc fusion, indicating that the anti-VEGFA antibody-Fc fusion protein had stronger target affinity and potentially superior efficacy in blocking the VEGFA signaling pathway.

The preferred embodiments of the present disclosure are described in detail above, which, however, are not intended to limit the present disclosure. Within the scope of the technical conception of the present disclosure, various simple modifications can be made to the technical solutions of the present disclosure, all of which will fall within the protection scope of the present disclosure.

In addition, it should be noted that the various specific technical features described in the above specific embodiments can be combined in any suitable manner in which the features do not contradict each other. In order to avoid unnecessary repetition, such combinations will not be illustrated separately.

**Claims**

1. An anti-VEGFA antibody or an antigen-binding fragment thereof, comprising CDR-H1, CDR-H2, and CDR-H3 of a heavy chain variable region, wherein

   an amino acid sequence of the CDR-H1 comprises SYTMG (SEQ ID NO: 1) or an amino acid sequence having at least 80% identity to SYTMG (SEQ ID NO: 1);
   an amino acid sequence of the CDR-H2 comprises AISKGGYKYX$_1$X$_2$VSLEA (SEQ ID NO: 2) or an amino acid sequence having at least 80% identity to AISKGGYKYX$_1$X$_2$VSLEA (SEQ ID NO: 2);
   an amino acid sequence of the CDR-H3 comprises TRAYGSSRLX$_3$LAX$_4$TYEY (SEQ ID NO: 3) or an amino acid sequence having at least 80% identity to TRAYGSSRLX$_3$LAX$_4$TYEY (SEQ ID NO: 3).

2. The anti-VEGFA antibody or the antigen-binding fragment thereof according to claim 1, wherein X$_1$X$_2$ in SEQ ID NO: 2 represents DS, DA, NT, DT, NA, or NS; X$_3$ in SEQ ID NO: 3 represents R or K, and X$_4$ in SEQ ID NO: 3 represents D, N, E, or K.

3. The anti-VEGFA antibody or the antigen-binding fragment thereof according to claim 1 or 2, wherein the amino acid sequences of the CDR-H1, the CDR-H2, and the CDR-H3 comprise any one of the following groups:

   A) SEQ ID NOs: 1, 4, and 9;
   B) SEQ ID NOs: 1, 5, and 9;
   C) SEQ ID NOs: 1, 6, and 10;
   D) SEQ ID NOs: 1, 4, and 11;
   E) SEQ ID NOs: 1, 7, and 12;
   F) SEQ ID NOs: 1, 6, and 11;
   G) SEQ ID NOs: 1, 4, and 10;
   H) SEQ ID NOs: 1, 5, and 12;
   I) SEQ ID NOs: 1, 4, and 12;
   J) SEQ ID NOs: 1, 8, and 12; and
   K) SEQ ID NOs: 1, 33, and 34.

4. The anti-VEGFA antibody or the antigen-binding fragment thereof according to any one of claims 1-3, comprising a humanized sequence, wherein an engineered site of the humanized sequence is located in a non-CDR region, and preferably, the engineered site of the humanized sequence is located in a framework region and/or a constant region of the antibody.

5. The anti-VEGFA antibody or the antigen-binding fragment thereof according to any one of claims 1-4, wherein the anti-VEGFA antibody or the antigen-binding fragment thereof is a nanobody, a chimeric antibody, a Fab fragment, a Fab' fragment, a Fd fragment, a Fd' fragment, a Fv fragment, a dAb fragment, an isolated CDR, a F(ab')$_2$ fragment, a single domain antibody, a single chain antibody molecule, or a linear antibody.

6. The anti-VEGFA antibody or the antigen-binding fragment thereof according to any one of claims 1-5, wherein an amino acid sequence of the anti-VEGFA antibody or the antigen-binding fragment thereof comprises any one of the amino acid sequences of SEQ ID NOs: 13-32, 35-61, and 64-67, or has at least 80% identity to any one of the amino acid sequences of SEQ ID NOs: 13-32, 35-61, and 64-67.

7. The anti-VEGFA antibody or the antigen-binding fragment thereof according to any one of claims 1-6, wherein the anti-VEGFA antibody or the antigen-binding fragment thereof binds to a human or monkey VEGFA protein.

8. A fusion protein, comprising the anti-VEGFA antibody or the antigen-binding fragment thereof according to any one of claims 1-7.

9. The fusion protein according to claim 8, further comprising, in addition to the anti-VEGFA antibody or the antigen-binding fragment thereof according to any one of claims 1-7, an additional anti-VEGFA antibody or an antigen-binding fragment thereof, or an antibody against an additional target or an antigen-binding fragment thereof, or an additional functional component, wherein

preferably, the additional functional component comprises, but is not limited to, one or a combination of two or more elements selected from serum albumin, a cytokine, transferrin, a scaffold protein, an oligopeptide, an oligopeptide polymer, a polypeptide, a polypeptide polymer, a polysaccharide, a fatty chain, avidin, biotin, streptavidin, a toxin, a drug, a nucleic acid, a radionuclide and a marker thereof, a PEGylated ingredient, and a Fc fragment;

preferably, the additional target is selected from IgG, VEGFB, VEGFC, VEGFD, VEGFR, FGF, FGFR, PIGF, PDGF, ANG2, Endoglin (CD105), TGF, Integrin, Integrin receptors, interleukins (e.g., IL-1$\beta$, IL-2, IL-3, IL-4, IL-6, IL-10, IL-12, IL-15, IL-17, IL-23, etc.), interleukin receptors (e.g., IL1R1, IL2R$\alpha$, IL3R, IL4R$\alpha$, IL6R, IL10R, IL12R, IL15R$\alpha$, IL17R, IL23R, etc.), PCSK9, TNF-$\alpha$, TNFR, RANKL, complement protein C3, complement protein C5, G protein-coupled receptors (GPCRs), GLP1R, CD3, CD19, CD20, CD22, CD25, CD27, CD28, CD30, CD33, CD38, CD40, CD47, CD80, CD86, CD96, CD99, CD111, CD112, CD123, CD133, CD138, CD155, CD171, Claudin 18.2, OX40, ICOS, CTLA4, 4-1BB, TCR, B7-1, B7-2, BTLA, TIM-3, LAG3, Galectin-9, PD-L1, PD-L2, PD-1, TIGIT, EGFR, Her2, PSCA, CEA, FAP, EGFRVIII, BCMA, PSMA, CA125, EphA2, C-met, L1CAM, CS1, ROR1, EC, NY-ESO-1, MUC1, MUC16, mesothelin, LewisY, GPC3, GD2, EPG, DLL 3, and 5T4.

10. A chimeric antigen receptor, wherein an extracellular domain of the chimeric antigen receptor comprises the anti-VEGFA antibody or the antigen-binding fragment thereof according to any one of claims 1-7.

11. An immune cell expressing the anti-VEGFA antibody or the antigen-binding fragment thereof according to any one of claims 1-7 or the chimeric antigen receptor according to claim 10.

12. A nucleic acid encoding the anti-VEGFA antibody or the antigen-binding fragment thereof according to any one of claims 1-7 or encoding the fusion protein according to any one of claims 8-9.

13. A vector, comprising the nucleic acid according to claim 12.

14. A host cell, comprising the nucleic acid according to claim 12 or the vector according to claim 13.

15. A preparation method for the anti-VEGFA antibody or the antigen-binding fragment thereof according to any one of claims 1-7, comprising introducing a nucleic acid or vector encoding the anti-VEGFA antibody or the antigen-binding fragment thereof into a host cell, and subsequently inducing expression thereof.

16. A method for screening the anti-VEGFA antibody or the antigen-binding fragment thereof according to any one of claims 1-7, comprising immunizing an alpaca with a human or monkey VEGFA protein.

17. A product for treating and/or diagnosing a disease, comprising any one of the following:

A) the anti-VEGFA antibody or the antigen-binding fragment thereof according to any one of claims 1-7;
B) the fusion protein according to any one of claims 8-9;
C) the chimeric antigen receptor according to claim 10;
D) the immune cell according to claim 11;
E) the nucleic acid according to claim 12;
F) the vector according to claim 13; or
G) the host cell according to claim 14.

18. Use of the anti-VEGFA antibody or the antigen-binding fragment thereof according to any one of claims 1-7, the fusion protein according to any one of claims 8-9, the chimeric antigen receptor according to claim 10, the immune cell according to claim 11, the nucleic acid according to claim 12, the vector according to claim 13, or the host cell according to claim 14 in preparing a product for treating and/or preventing a VEGFA-associated disease, or in preparing a product for blocking VEGFA-mediated vascular endothelial cell proliferation or inhibiting angiogenesis, or in preparing an antibody-drug conjugate or an antibody diagnostic kit or a tracer.

19. A method for detecting VEGFA, comprising contacting a sample to be tested with the anti-VEGFA antibody or the antigen-binding fragment thereof according to any one of claims 1-7, and subsequently detecting the content of a complex formed by VEGFA and the anti-VEGFA antibody or the antigen-binding fragment thereof.

20. A method for treating and/or preventing a disease, comprising administering to an individual the anti-VEGFA antibody or the antigen-binding fragment thereof according to any one of claims 1-7, the fusion protein according to any one of claims 8-9, the chimeric antigen receptor according to claim 10, the immune cell according to claim 11, the nucleic acid according to claim 12, the vector according to claim 13, the host cell according to claim 14, or the product for treating and/or diagnosing a disease according to claim 17.

21. A method for treating and/or preventing a disease, comprising contacting the anti-VEGFA antibody or the antigen-binding fragment thereof according to any one of claims 1-7, the fusion protein according to any one of claims 8-9, the chimeric antigen receptor according to claim 10, the immune cell according to claim 11, the nucleic acid according to claim 12, the vector according to claim 13, the host cell according to claim 14, or the product for treating and/or diagnosing a disease according to claim 17 with a target cell.

22. The method according to claim 21, wherein the target cell is selected from cells expressing VEGFA, such as cardiomyocytes, renal proximal tubule cells, hepatocytes, vascular endothelial cells, granulosa cells, specialized epithelial cells, mesenchymal cells, macrophages, platelets, dendritic cells, activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells, tumor cells, etc.

23. The method according to any one of claims 20-22, wherein the disease is a disease associated with the VEGFA signaling pathway; preferably, the disease is selected from a tumor, abnormal vascular proliferation, and an ophthalmic disease involving angiogenesis (e.g., a fundus vascular disease).

24. A method for blocking VEGF A-mediated vascular endothelial cell proliferation or inhibiting angiogenesis, comprising contacting a vascular endothelial cell with the anti-VEGFA antibody or the antigen-binding fragment thereof according to any one of claims 1-7, the fusion protein according to any one of claims 8-9, the chimeric antigen receptor according to claim 10, the immune cell according to claim 11, the nucleic acid according to claim 12, the vector according to claim 13, the host cell according to claim 14, or the product for treating and/or diagnosing a disease according to claim 17.

FIG. 1

FIG. 2

FIG. 3

FIG. 4

FIG. 5

ELISA : Mouse VEGFA164 binding

FIG. 6

ELISA : Rat VEGFA164 binding

FIG. 7

(A) Humanized antibodies of V1

1. BI-VEGF ab
2. V1-DP
3. V1-SA1
4. V1-3m
5. V1-SA3

(B) Humanized antibodies of V13  Humanized antibodies of V15

1. V13-2m
2. V13-4m
3. V15-2m
4. V15-4m

(C) Humanized antibodies of V30  Humanized antibodies of V40

1. V30-2H-78
2. V30-2H-87
3. V30-2H-2m
4. V30-3H-2m

5. V40-3H-2m
6. V40-3H-4m
7. V40-4H-2m

(D) Humanized antibodies of V43

1. V43-1H-78
2. V43-1H-87
3. V43-1H-2m
4. V43-3H-78
5. V43-3H-87

FIG. 8

FIG. 9

FIG. 10

FIG. 11

**V1-SA1; VEGFA165**

FIG. 12

Tm

FIG. 13

FIG. 14

1. V15-PPCP-Fc, non-reducing
2. V15-PPCP-Fc, reducing

1. V15-9GS-PPCP-Fc, reducing
2. V15-Fc, reducing

FIG. 15

FIG. 16

**V1-SA1-Fc-m1; VEGFA165**

FIG. 17

## INTERNATIONAL SEARCH REPORT

| International application No. |
|---|
| **PCT/CN2023/118600** |

### A. CLASSIFICATION OF SUBJECT MATTER

C07K16/22(2006.01)i; A61K39/395(2006.01)i

According to International Patent Classification (IPC) or to both national classification and IPC

### B. FIELDS SEARCHED

Minimum documentation searched (classification system followed by classification symbols)

IPC:C07K,A61K

Documentation searched other than minimum documentation to the extent that such documents are included in the fields searched

Electronic data base consulted during the international search (name of data base and, where practicable, search terms used)

CJFD, CNTXT, ENTXT, ENTXTC, STN, PUBMED, SpringerLink, ISI Web of Knowledge, Elsevier Science Direct, Wiley InterScience, Nature, Science, CNKI, 万方, WANFANG, NCBI: SEQ ID NO: 1-32, 35-61, 63-67, VEGF, antibody, vhh, 血管内皮生长因子.

### C. DOCUMENTS CONSIDERED TO BE RELEVANT

| Category* | Citation of document, with indication, where appropriate, of the relevant passages | Relevant to claim No. |
|---|---|---|
| X | CN 103562222 A (BOEHRINGER INGELHEIM INTERNATIONAL GMBH) 05 February 2014 (2014-02-05) <br> claims 3-40, description paragraphs 235, 246-329, table 3, SEQ ID NOs: 2-57 | 1-2, 4-24 |
| X | CN 105037542 A (BOEHRINGER INGELHEIM INTERNATIONAL GMBH) 11 November 2015 (2015-11-11) <br> claims 1-13, description paragraphs 195-266, table 57, SEQ 54-62 | 1-2, 4-24 |
| X | CN 103209995 A (BOEHRINGER INGELHEIM INTERNATIONAL GMBH) 17 July 2013 (2013-07-17) <br> claims 2-24, description paragraphs 184, 192-270, figures 17-19, table 2 | 1-2, 4-24 |
| X | CN 113546178 A (THE REGENTS OF THE UNIVERSITY OF CALIFORNIA) 26 October 2021 (2021-10-26) <br> description paragraphs 26-27, 33, 99-100, SEQ ID NO: 6 | 1-2, 4-24 |
| A | WO 2022175474 A1 (DOTBIO PTE. LTD.) 25 August 2022 (2022-08-25) <br> entire document | 1-24 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 December 2023** | **13 December 2023** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/118600** |

**C. DOCUMENTS CONSIDERED TO BE RELEVANT**

| Category* | Citation of document, with indication, where appropriate, of the relevant passages | Relevant to claim No. |
| --- | --- | --- |
| A | WO 2021013065 A1 (SINOCELLTECH LTD.) 28 January 2021 (2021-01-28)<br>entire document | 1-24 |
| A | KHODABAKHSH, F. et al. "Development of a Novel Nano-Sized Anti-VEGFA Nanobody with Enhanced Physicochemical and Pharmacokinetic Properties"<br>*Artificial Cells, Nanomedicine, and Biotechnology,*<br>Vol. 46, No. (7), 25 August 2017 (2017-08-25), 1402-1414<br>entire document | 1-24 |

Form PCT/ISA/210 (second sheet) (July 2022)

## INTERNATIONAL SEARCH REPORT

International application No.

**PCT/CN2023/118600**

**Box No. I     Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

         ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

| International application No. |
|---|
| **PCT/CN2023/118600** |

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☑ Claims Nos.: **20-24**
   because they relate to subject matter not required to be searched by this Authority, namely:

   Claims 20-24 relate to a method for treating and/or preventing a disease, which falls within methods for treatment of diseases as defined in PCT Rule 39.1(iv). However, a search is still performed on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2023/118600**

| Patent document cited in search report | | | Publication date (day/month/year) | Patent family member(s) | | | Publication date (day/month/year) |
|---|---|---|---|---|---|---|---|
| CN | 103562222 | A | 05 February 2014 | CY | 1118339 | T1 | 28 June 2017 |
| | | | | MX | 343440 | B | 07 November 2016 |
| | | | | SG | 193561 | A1 | 29 November 2013 |
| | | | | AU | 2017200237 | A1 | 02 February 2017 |
| | | | | AU | 2017200237 | B2 | 17 May 2018 |
| | | | | HK | 1190412 | A1 | 04 July 2014 |
| | | | | ECSP | 13013001 | A | 31 December 2013 |
| | | | | HUE | 030148 | T2 | 28 April 2017 |
| | | | | PT | 2694546 | T | 28 December 2016 |
| | | | | AP | 201307085 | D0 | 31 August 2013 |
| | | | | CA | 2827817 | A1 | 04 October 2012 |
| | | | | CA | 2827817 | C | 15 December 2020 |
| | | | | PE | 20140448 | A1 | 13 April 2014 |
| | | | | RS | 55361 | B1 | 31 March 2017 |
| | | | | EA | 201301108 | A1 | 31 March 2014 |
| | | | | EA | 025148 | B1 | 30 November 2016 |
| | | | | JP | 2014515602 | A | 03 July 2014 |
| | | | | JP | 6023786 | B2 | 09 November 2016 |
| | | | | HK | 1225400 | A1 | 08 September 2017 |
| | | | | ES | 2606302 | T3 | 23 March 2017 |
| | | | | SI | 2694546 | T1 | 31 January 2017 |
| | | | | WO | 2012131078 | A1 | 04 October 2012 |
| | | | | JP | 2017023152 | A | 02 February 2017 |
| | | | | JP | 6297657 | B2 | 20 March 2018 |
| | | | | AP | 201307085 | A0 | 31 August 2013 |
| | | | | MY | 171007 | A | 23 September 2019 |
| | | | | BR | 112013025304 | A2 | 06 June 2017 |
| | | | | BR | 112013025304 | B1 | 11 October 2022 |
| | | | | EP | 2694546 | A1 | 12 February 2014 |
| | | | | EP | 2694546 | B1 | 21 September 2016 |
| | | | | MA | 34979 | B1 | 01 March 2014 |
| | | | | TN | 2013000390 | A1 | 20 January 2015 |
| | | | | AU | 2012237234 | A1 | 05 September 2013 |
| | | | | AU | 2012237234 | B2 | 20 October 2016 |
| | | | | EP | 3144322 | A2 | 22 March 2017 |
| | | | | EP | 3144322 | A3 | 31 May 2017 |
| | | | | AR | 085984 | A1 | 13 November 2013 |
| | | | | HRP | 20161689 | T1 | 24 February 2017 |
| | | | | US | 2022017642 | A1 | 20 January 2022 |
| | | | | EA | 201600338 | A1 | 30 September 2016 |
| | | | | EA | 036746 | B1 | 16 December 2020 |
| | | | | CL | 2013002623 | A1 | 14 February 2014 |
| | | | | NZ | 614249 | A | 29 May 2015 |
| | | | | US | 2020010569 | A1 | 09 January 2020 |
| | | | | US | 11161916 | B2 | 02 November 2021 |
| | | | | KR | 20140018317 | A | 12 February 2014 |
| | | | | KR | 101907572 | B1 | 15 October 2018 |
| | | | | DK | 2694546 | T3 | 05 December 2016 |
| | | | | PL | 2694546 | T3 | 31 March 2017 |
| | | | | MX | 2013010949 | A | 06 June 2014 |

Form PCT/ISA/210 (patent family annex) (July 2022)

# EP 4 582 447 A1

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| | | | | MX | 337543 | B | 10 March 2016 |
| | | | | SG | 10201602373 | TA | 30 May 2016 |
| | | | | IL | 227936 | A0 | 30 September 2013 |
| | | | | IL | 227936 | B | 28 June 2018 |
| | | | | UY | 33998 | A | 28 September 2012 |
| | | | | UA | 114707 | C2 | 25 July 2017 |
| | | | | US | 2013078248 | A1 | 28 March 2013 |
| | | | | US | 9527925 | B2 | 27 December 2016 |
| | | | | CO | 6801639 | A2 | 29 November 2013 |
| | | | | LT | 2694546 | T | 10 November 2016 |
| | | | | US | 2017247475 | A1 | 31 August 2017 |
| | | | | US | 10414828 | B2 | 17 September 2019 |
| CN | 105037542 | A | 11 November 2015 | MX | 2012003897 | A | 08 May 2012 |
| | | | | TW | 201124533 | A | 16 July 2011 |
| | | | | US | 2014120095 | A1 | 01 May 2014 |
| | | | | CA | 2775422 | A1 | 07 April 2011 |
| | | | | AP | 201206188 | A0 | 30 April 2012 |
| | | | | BR | 112012007239 | A2 | 24 September 2019 |
| | | | | EP | 2483314 | A1 | 08 August 2012 |
| | | | | JP | 2013506411 | A | 28 February 2013 |
| | | | | JP | 5833009 | B2 | 16 December 2015 |
| | | | | AP | 201206188 | D0 | 30 April 2012 |
| | | | | MA | 33607 | B1 | 01 September 2012 |
| | | | | NZ | 598956 | A | 25 July 2014 |
| | | | | JP | 2016026207 | A | 12 February 2016 |
| | | | | IL | 218542 | A0 | 31 May 2012 |
| | | | | UY | 32920 | A | 29 April 2011 |
| | | | | PE | 20121024 | A1 | 10 August 2012 |
| | | | | ECSP | 12011835 | A | 29 June 2012 |
| | | | | EA | 201200548 | A1 | 28 December 2012 |
| | | | | CL | 2012000826 | A1 | 19 October 2012 |
| | | | | IN | 2758 2012 | A | 18 September 2015 |
| | | | | NZ | 626302 | A | 25 September 2015 |
| | | | | US | 2011172398 | A1 | 14 July 2011 |
| | | | | KR | 20120101375 | A | 13 September 2012 |
| | | | | TN | 2012000145 | A1 | 19 September 2013 |
| | | | | AU | 2010302589 | A1 | 19 April 2012 |
| | | | | AR | 078515 | A1 | 16 November 2011 |
| | | | | WO | 2011039370 | A1 | 07 April 2011 |
| CN | 103209995 | A | 17 July 2013 | JP | 2013541939 | A | 21 November 2013 |
| | | | | JP | 5979504 | B2 | 24 August 2016 |
| | | | | IL | 224800 | B | 31 March 2020 |
| | | | | EP | 2611830 | A1 | 10 July 2013 |
| | | | | CA | 2808696 | A1 | 08 March 2012 |
| | | | | CA | 2808696 | C | 20 December 2016 |
| | | | | WO | 2012028716 | A1 | 08 March 2012 |
| | | | | US | 2020308267 | A1 | 01 October 2020 |
| | | | | EP | 2727939 | A2 | 07 May 2014 |
| | | | | EP | 2727939 | A3 | 21 May 2014 |
| | | | | KR | 20130101045 | A | 12 September 2013 |

Form PCT/ISA/210 (patent family annex) (July 2022)

# EP 4 582 447 A1

## INTERNATIONAL SEARCH REPORT
### Information on patent family members

International application No.

**PCT/CN2023/118600**

| Patent document cited in search report | | | Publication date (day/month/year) | Patent family member(s) | | | Publication date (day/month/year) |
|---|---|---|---|---|---|---|---|
| | | | | KR | 101844875 | B1 | 03 April 2018 |
| | | | | AR | 082892 | A1 | 16 January 2013 |
| | | | | NZ | 607394 | A | 24 December 2014 |
| | | | | UY | 33588 | A | 30 March 2012 |
| | | | | EA | 201300311 | A1 | 30 August 2013 |
| | | | | EA | 029277 | B1 | 30 March 2018 |
| | | | | MX | 2013002379 | A | 09 May 2013 |
| | | | | MX | 353813 | B | 31 January 2018 |
| | | | | US | 2016200806 | A1 | 14 July 2016 |
| | | | | US | 2022363744 | A1 | 17 November 2022 |
| | | | | US | 2018201671 | A1 | 19 July 2018 |
| | | | | CL | 2013000596 | A1 | 14 February 2014 |
| | | | | AU | 2011298281 | A1 | 14 March 2013 |
| | | | | AU | 2011298281 | B2 | 05 February 2015 |
| | | | | US | 2012225081 | A1 | 06 September 2012 |
| | | | | BR | 112013005151 | A2 | 04 August 2020 |
| CN | 113546178 | A | 26 October 2021 | WO | 2017100470 | A1 | 15 June 2017 |
| | | | | CA | 3022905 | A1 | 15 June 2017 |
| | | | | ES | 2925248 | T3 | 14 October 2022 |
| | | | | EP | 3386520 | A1 | 17 October 2018 |
| | | | | EP | 3386520 | A4 | 17 July 2019 |
| | | | | EP | 3386520 | B1 | 20 July 2022 |
| | | | | US | 2018318431 | A1 | 08 November 2018 |
| | | | | US | 10765759 | B2 | 08 September 2020 |
| | | | | US | 2021113702 | A1 | 22 April 2021 |
| | | | | US | 11723982 | B2 | 15 August 2023 |
| | | | | KR | 20180100569 | A | 11 September 2018 |
| WO | 2022175474 | A1 | 25 August 2022 | AR | 124917 | A1 | 17 May 2023 |
| | | | | CA | 3208368 | A1 | 25 August 2022 |
| | | | | CA | 3208389 | A1 | 25 August 2022 |
| | | | | AU | 2022223337 | A1 | 21 September 2023 |
| | | | | WO | 2022175481 | A1 | 25 August 2022 |
| | | | | AU | 2022222311 | A1 | 21 September 2023 |
| WO | 2021013065 | A1 | 28 January 2021 | US | 2022135665 | A1 | 05 May 2022 |
| | | | | JP | 2022537072 | A | 23 August 2022 |
| | | | | JP | 7229419 | B2 | 27 February 2023 |
| | | | | BR | 112022001021 | A2 | 12 April 2022 |
| | | | | AU | 2020316495 | A1 | 03 February 2022 |
| | | | | MX | 2022000780 | A | 14 February 2022 |
| | | | | KR | 20220034868 | A | 18 March 2022 |
| | | | | EP | 4043490 | A1 | 17 August 2022 |
| | | | | EP | 4043490 | A4 | 16 August 2023 |
| | | | | CA | 3150046 | A1 | 28 January 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9527925 B2